(19) Europäisches Patentamt
European Patent Office
Office européen des brevets

(11) **EP 1 729 738 B1**

(12) **EUROPEAN PATENT SPECIFICATION**

(45) Date of publication and mention
of the grant of the patent:
**01.07.2009 Bulletin 2009/27**

(51) Int Cl.:
***A61K 9/20*** (2006.01)

(86) International application number:
**PCT/US2005/007155**

(21) Application number: **05724660.5**

(22) Date of filing: **03.03.2005**

(87) International publication number:
**WO 2005/087203 (22.09.2005 Gazette 2005/38)**

(54) **POLYMERIC COMPOSITIONS AND DOSAGE FORMS COMPRISING THE SAME**

POLYMER-ZUSAMMENSETZUNGEN UND DIESE ENTHALTENDE DOSIERFORMEN

COMPOSITIONS POLYM RIQUES ET FORMES POSOLOGIQUES CONTENANT CES COMPOSITIONS

(84) Designated Contracting States:
**AT BE BG CH CY CZ DE DK EE ES FI FR GB GR HU IE IS IT LI LT LU MC NL PL PT RO SE SI SK TR**

(30) Priority: **05.03.2004 US 794660**
**05.03.2004 US 794659**
**05.03.2004 US 794448**
**05.03.2004 US 794449**

(43) Date of publication of application:
**13.12.2006 Bulletin 2006/50**

(73) Proprietor: **McNeil-PPC, Inc.**
**Skillman, NJ 08558 (US)**

(72) Inventors:
• HUANG, Hai, Yong
  **Princeton, New Jersey 08540 (US)**
• LEE, Der-Yang
  **Flemington, New Jersey 08822 (US)**
• LI, Shun, Por
  **Lansdale, Pennsylvania 19446 (US)**

(74) Representative: **Kirsch, Susan Edith**
**Carpmaels & Ransford**
**43 Bloomsbury Square**
**London WC1A 2RA (GB)**

(56) References cited:
US-A- 4 892 739    US-A1- 2002 064 556
US-B1- 6 790 459

**Description**

BACKGROUND OF THE INVENTION

1. Field of the Invention

This invention relates to polymeric compositions, and dosage forms such as modified release pharmaceutical compositions, comprising the same. This invention further relates to methods of providing predetermined active ingredient concentrations, which may be substantially constant or substantially non-constant, over an extended period of time, using such dosage forms. More particularly, this invention relates to modified release dosage forms for delivering one or more active ingredients in a controlled or delayed manner upon contacting of the dosage form with a liquid medium. The dosage form contains at least one active ingredient, and has a core and a shell. At least a portion of the shell is semipermeable to a liquid medium such as the gastro-intestinal (GI) fluids of a patient, such that the liquid medium diffuses through the semipermeable shell or shell portion to the core, for example due to osmosis. The shell or shell portion also provides for delivery of active ingredient to the liquid medium outside of the dosage form after the dosage form is contacted with the liquid medium.

2. Background Information

Modified release pharmaceutical dosage forms have long been used to optimize drug delivery and enhance patient compliance, especially by reducing the number of doses of medicine the patient must take in a day. For this purpose, it is often desirable to modify the rate of release of a drug (one particularly preferred type of active ingredient) from a dosage form into the GI fluids of a patient, especially to slow the release to provide prolonged action of the drug in the body.

The rate at which an orally delivered pharmaceutical active ingredient reaches its site of action in the body depends on a number of factors, including the rate and extent of drug absorption through the GI mucosa. To be absorbed into the circulatory system (blood), the drug must first be dissolved in the GI fluids. For many drugs, diffusion across the GI membranes is relatively rapid compared to dissolution. In these cases, the dissolution of the active ingredient is the rate limiting step in drug absorption, and controlling the rate of dissolution allows the formulator to control the rate of drug absorption into the circulatory system of a patient.

An important objective of modified release dosage forms is to provide a desired blood concentration versus time (pharmacokinetic, or PK) profile for the drug. Fundamentally, the PK profile for a drug is governed by the rate of absorption of the drug into the blood, and the rate of elimination of the drug from the blood. The type of PK profile desired depends, among other factors, on the particular active ingredient, and physiological condition being treated.

It is also particularly desirable for a pharmaceutical dosage form to deliver more than one drug at different rates from one another. Because the onset and duration of the therapeutic efficacy of drugs vary widely, as do their absorption, distribution, metabolism, and elimination, it is often desirable to modify the release of different drugs in different ways, or to have a first active ingredient immediately released from the dosage form, while a second drug is released in a delayed, controlled, sustained, prolonged, extended, or retarded manner.

Well known mechanisms by which a dosage form (or drug delivery system) can deliver drug at a controlled rate (e.g. sustained, prolonged, extended or retarded release) include diffusion, erosion, and osmosis. It is often practical to design dosage forms which use a combination of the above mechanisms to achieve a particularly desirable release profile for a particular active ingredient. It will be readily recognized by those skilled in the art that a dosage form construct which offers multiple compartments, such as for example multiple core portions and/or multiple shell portions, is particularly advantageous for its flexibility in providing a number of different mechanisms for controlling the release of one or more active ingredients.

In various embodiments of this invention, the modified release dosage forms of this invention act as osmotically controlled drug delivery systems. As discussed, for example, in Verma et al., "Osmotically Controlled Oral Drug Delivery," Drug Development and Industrial Pharmacy, 26(7), pp. 695-708 (2000), osmotically controlled drug delivery systems offer a number of advantages over conventional controlled release delivery systems. The basic principle of operation of osmotic drug delivery systems employs the difference in osmotic pressure between the exterior and interior of a dosage form which contains active ingredient. For example, as discussed by Verma et al., in an elementary osmotic pump (EOP) system, an active ingredient, together with a suitable osmotic solute is contained within a core such as a compressed tablet, which is coated at least in part with a semipermeable membrane or shell having a small orifice therein. When this dosage form contacts a liquid medium such as the aqueous environment of the GI tract, the active ingredient draws liquid through the semipermeable membrane due to the osmotic pressure gradient and forms a saturated solution of active ingredient within the dosage form. Hydrostatic pressure thus develops within the dosage form, and is relieved by the flow of saturated solution through the orifice and out of the dosage form, thereby delivering the active ingredient to the patient until the pressures inside and outside the dosage form are equal. Such a system is exemplified in U.S. Patent No. 3,845,770, which discloses (see Fig. 1 of U.S. Patent No. 3,845,770) a device having a semipermeable wall which surrounds a compartment containing active ingredient. A passageway communicates with the compartment and the exterior of the device. Fluid permeates the wall into the compartment and produces a solution of active ingredient which is released through the passageway.

Various osmotic drug delivery systems are well known in the art and are disclosed in, for example, U.S. Patent No. 3,995,631, U.S. Patent No. 4,111,202, U.S. Patent No. 4,327,725, U.S. Patent No. 4,449,983, U.S. Patent No. 4,627,971, U.S. Patent No. 4 892 739, U.S. Patent No. 5,830,501, U.S. Patent No. 6,342,249, and European Patent Publication No. 0384642.

The modified release dosage forms of this invention employ a core and a shell, which may optionally comprise multiple portions having different compositions and/or functions. The dosage forms of this invention are prepared by a novel method which enables at least portion of the shell to be semipermeable. In contrast, current core-shell systems are limited by the available methods for manufacturing them, as well as the materials that are suitable for use with the current methods. A shell, or coating, which co nfers modified release properties is typically applied via conventional methods, such as for example, spray-coating in a coating pan. Pan-coating produces a single shell which essentially surrounds the core. The single shell is inherently limited in its functionality. It is possible via pan-coating to apply multiple concentric shells, each with a different functionality, however such systems are limited in that the outer shell must first dissolve before the functionality conferred by each successive layer can be realized. It is also known, via pan coating, to deliver a first dose of active ingredient from a coating, and a second dose of active ingredient from a core. Dosage forms having sprayed coatings which provide delayed release are described, for example, in Maffione et al., "High-Viscosity HPMC as a Film-Coating Agent," Drug Development and Industrial Pharmacy (1993) 19(16), pp. 2043-2053. U.S. Patent No. 4,576,604, for example, discloses an osmotic device (dosage form) comprising a drug co mpartment surrounded by a wall (coating) in which the coating may comprise an immediate release dose of drug, and the inner drug compartment may comprise a sustained release dose of drug. The coating compositions that can be applied via spraying are limited by their viscosity. High viscosity solutions are difficult or impractical to pump and deliver through a spray nozzle. Spray coating methods suffer the further limitations of being time-intensive and costly. Several hours of spraying may be required to spray an effective amount of coating to control the release of an active ingredient. Coating times of 8 to 24 hours are not uncommon.

It is one object of this invention to provide a novel polymeric composition suitable for coating dosage forms, such as osmotic modified release dosage forms. It is another object of this invention to provide a modified release dosage form in which at least one active ingredient contained therein exhibits a modified release profile upon contacting of the dosage form with a liquid medium. It is one feature of the dosage form of this invention that it has a semipermeable shell or shell portion. It is another feature of this invention that the semipermeable shell or shell portion allows the liquid medium to diffuse through the shell or shell portion to the core, for example due to osmosis and permeate into the core. It is another feature of this invention that the shell provides for delivery of liquid medium carrying active ingredient out of the dosage form. Other objects, features and advantages of the invention will be apparent to those skilled in the art from the detailed description set forth below.

SUMMARY OF THE INVENTION

The invention provides a composition comprising, consisting of, and/or consisting essentially of, based upon the total wet weight of the composition: a) from about 5 percent to about 50 percent of a water insoluble, film forming polymer; b) from about 0.05 percent to about 15 percent of a gelling agent; and c) from about 40 percent to about 95 percent of a multisolvent system, wherein said multisolvent system is comprised of, based upon the total weight of the multisolvent system, from about 10 percent to about 50 percent of water and from about 50 percent to about 90 percent of a water miscible organic solvent.

The invention further provides a composition comprising, consisting of, and/or consisting essentially of, based upon the total dry weight of the composition: a) from about 40 percent to about 99 percent of a water insoluble, film forming polymer; and b) from about 1 percent to about 30 percent of a gelling agent.

This invention further provides a dosage form comprising (a) at least one active ingredient; (b) a core having an outer surface; and (c) a shell which resides upon at least a portion of the core outer surface, wherein at least a portion of the shell is semipermeable, the shell is comprised of, based upon the total dry weight of the shell, from about 40 percent to about 99 percent of a water insoluble film forming polymer; and from about 1 percent to about . 30 percent of a gelling agent, wherein said shell has means for providing the active ingredient to a liquid medium outside the shell after contacting of the dosage form with the liquid medium.

The invention also provides a dosage form comprising (a) at least one active ingredient; (b) a core having an outer surface; and (c) a shell which resides upon at least a portion of the core outer surface, wherein the shell comprises a first shell portion which is semipermeable to the liquid medium, and a second shell portion which is compositionally different than the first shell portion, the first and second shell portions each are substantially in contact with the core outer surface, and said first portion may be comprised of, for e.g., based upon the total dry weight of the first portion, from about 40 percent to about 99 percent of a water insoluble film forming polymer, and from about 1 percent to about 30 percent of a gelling agent, wherein the shell has means for providing the active ingredient to a liquid medium outside the shell after contacting of the dosage form with the liquid medium, such as, for e.g., a passageway through the first shell portion and/or the second shell portion.

The invention further provides a dosage form comprising: (a) at least one active ingredient; (b) a core having an outer surface, a first core portion, a second core portion, and a third core portion located between the first and second core portions, wherein the third core portion comprises an osmopolymer; (c) and a shell which resides upon at least a portion of the core outer surface, in which the shell comprises a first shell portion which is semipermeable to the liquid medium and may be comprised of, for e.g., based upon the total dry weight of the first portion, from about 40 percent to about 99 percent of a water insoluble film forming polymer; and from about 1 percent to about 30 percent of a gelling agent, and a second shell portion which is compositionally different than the first shell portion, the first and second shell portions each are substantially in contact with the core outer surface, and at least one of the first or second shell portions has at least one passageway therein extending to the core outer surface.

The invention also provides a dosage form comprising (a) at least one active ingredient; (b) a core having an outer surface; (c) and a shell which resides upon at least a portion of the core outer surface, in which the shell comprises a first shell portion which is semipermeable to the liquid medium and is comprised of, based upon the total dry weight of the first portion, from about 40 percent to about 99 percent of a water insoluble film forming polymer; and from about 1 percent to about 30 percent of a gelling agent, and a second shell portion which is optionally compositionally different than the first shell portion, the first and second shell portions each are substantially in contact with the core outer surface, and the shell and core have a continuous cavity therein defining an interior surface, wherein neither the first shell portion nor the second shell portion extend substantially upon the interior surface.

The invention further provides a dosage form comprising: (a) at least one active ingredient; (b) a core having an outer surface, a first core portion, a second core portion, and a third core portion located between the first and second core portions, wherein the third core portion comprises a osmopolymer; and (c) a shell which resides upon at least a portion of the core outer surface, in which the shell comprises a first shell portion which is semipermeable to the liquid medium and may be comprised of, for e.g., from about 40 percent to about 99 percent of a water insoluble film forming polymer; and from about 1 percent to about 30 percent of a gelling agent, and a second shell portion which is compositionally different than the first shell portion, the first and second shell portions each are substantially in contact with the core outer surface, and the shell and core have a continuous cavity therein defining an interior surface, wherein neither the first shell portion nor the second shell portion extend substantially upon the interior surface.

BRIEF DESCRIPTION OF THE DRAWINGS

Figure 1A depicts a cross-sectional side view of one embodiment of the dosage form of this invention.

Figure 1 B depicts a cross-sectional side view of another embodiment of the dosage form of this invention.

Figure 2 depicts a cross-sectional side view of another embodiment of the dosage form of this invention.

Figure 3 depicts a cross-sectional side view of another embodiment of the dosage form of this invention.

Figure 4 depicts a cross-sectional side view of another embodiment of the dosage form of this invention.

Figure 5 depicts a cross-sectional side view of another embodiment of the dosage form of this invention.

Figure 6 depicts a cross-sectional side view of another embodiment of the dosage form of this invention.

Figures 7A and 7B depict cross-sectional micrographs of a dosage form coated with a prior art coating composition.

Figure 8A and 8B depict cross-sectional micrographs of a dosage form coated with an embodiment of this invention.

Figure 9 depicts the release profile of active ingredient for one embodiment of the dosage form of this invention, as analyzed via the dissolution test described in Example 5.

Figures 10 -13, respectively, depict the results of tensile testing of films formed from compositions described in Tables E, F, G, and H, respectively.

DETAILED DESCRIPTION OF THE INVENTION

It is believed that one skilled in the art can, based upon the description herein, utilize the present invention to its fullest extent. The following specific embodiments are to be construed as merely illustrative, and not limitative of the remainder of the disclosure in any way whatsoever.

Unless defined otherwise, all technical and scientific terms used herein have the same meaning as commonly understood by one of ordinary skill in the art to which the invention belongs. Also, all publications, patent applications, patents, and other references mentioned herein are incorporated by reference. As used herein, all percentages are by weight unless otherwise specified.

As used herein, the term "dosage form" applies to any solid object, semi-solid, or liquid composition designed to contain a specific pre-determined amount (dose) of a certain ingredient, for example an active ingredient as defined below. Suitable dosage forms include pharmaceutical drug delivery systems, including those for oral administration, buccal administration, rectal administration, topical or mucosal delivery, or subcutaneous implants, or other implanted drug delivery systems; or compositions for delivering minerals, vitamins and other nutraceuticals, oral care agents, flavorants, and the like. The dosage forms of the present invention are typically considered to be solid; however, they may contain liquid or semi-solid components. In one embodiment, the dosage form is an orally administered system for delivering a pharmaceutical active ingredient to the GI tract of a human.

The dosage forms of this invention exhibit modified release of one or more active ingredients contained therein. The active ingredient or ingredients may be found within the core, the shell, the overcoating, or a portion or combination thereof. As used herein, the term "modified release" shall apply to dosage forms, coatings, shells, cores, portions thereof, or compositions that alter the release of an active ingredient in any manner. The active ingredient or ingredients that are released in a modified manner may be contained within the coating, shell, core, composition, or portion thereof providing the modification. Alternatively the modified release active ingredient may be contained in a different portion of the dosage form from the coating, shell, core, composition, or portion thereof providing the modification; for example the modified release active ingredient may be contained in a core portion, and the modification may be provided by the overlaying shell portion. Types of modified release include controlled, prolonged, sustained, extended, delayed, pulsatile, repeat action, and the like. Suitable mechanisms for achieving these types of modified release include diffusion, erosion, surface area control via geometry and/or impermeable barriers, or other mechanisms known in the art. Moreover, the modified release properties of the dosage form may be achieved through design of the core or a portion thereof, or the shell or portion thereof, or a combination of two or more of these parts of the dosage form.

The dissolution profile of each active ingredient from the dosage form may be governed by a sum of contributions from the properties of the various portions. Additionally, a single portion, such as for example a core portion, may possess a combination of erosional and diffusional properties. In any case, the dissolution rate of a particular active ingredient from the dosage form will be the sum of the contributions from all the various mechanisms contributed by the various portions of the dosage form which effect the release of that particular active ingredient

The dosage forms of the present invention are designed to release substantially all (i.e., e.g. at least about 80%, or at least about 90%, or at least about 95%) of the active ingredient contained therein, within a specified amount of time. As used herein, the total amount of time required for substantially all of the active ingredient(s) to be released from the dosage form shall be referred to as the "dosing interval." During the dosing interval, the amount of drug released is typically measured at several time points.

As used herein, the term "time interval" shall refer to periods of time during the dosing interval, over which a periodic rate of release may be measured. The time interval may be the entire dosing interval, or a portion thereof.

As used herein, a drug "release rate" refers to the quantity of drug released from a dosage form per unit time, e.g., milligrams of drug released per hour (mg/hr). Drug release rates are calculated under *in vitro* dosage form dissolution testing conditions known in the art. As used herein, a drug release rate obtained at a specified time "following administration" refers to the *in vitro* drug release rate obtained at the specified time following implementation of an appropriate dissolution test.

As used herein, a "periodic release rate" refers to the quantity per unit time of drug released from a dosage form during a specified periodic interval as determined at the end of that specified periodic interval, i.e., at each periodic interval when a determination is made, the quantity per unit time of drug released represents the periodic release rate during that periodic interval. For example, the quantity of drug released per hour (h) determined as the difference in quantity released between t=0 and t=2h divided by the time interval of 2 hours represents the periodic release rate during the first two hours following administration, the quantity of drug released per hour as determined from t=2h to t=4h represents the periodic release rate from two to four hours following administration, etc.

As used herein, a "constant release rate" is obtained over a given time interval when the periodic release rates determined during two or more portions of the time interval are substantially the same, i.e. not more than 6% different. As used herein, "non-constant release rate" shall mean two or more periodic release rates are not the same, i.e. more than 6% different, over the entire duration of the specified interval.

As used herein, an "ascending release rate" refers to a periodic release rate that is increased over the immediately preceding periodic release rate. For example, when the quantity of drug released from a dosage form is measured at hourly intervals and the quantity of drug released during the fifth hour following administration is greater than the quantity of drug released from the dosage form during the fourth hour following administration, an ascending release rate from the fourth hour to the fifth hour has occurred. It will be appreciated that the first periodic release rate measured, e.g., the periodic release rate at t=1 hour (unless equal to 0), will always be greater than the release rate during the preceding period, e.g., the hour before the dosage form was administered, and thus, the first periodic release rate always constitutes an occurrence of an ascending release rate.

As used herein, "ascending blood level" refers to the PK profile obtained when the rate of release of drug from the dosage form, and also its absorption into the bloodstream, exceeds its rate of elimination from the blood of a mammal for a period of time, producing an increasing blood level over the course of the dosing interval or a portion thereof.

As used herein, a "burst release" profile refers to a release profile which meets immediate release criteria during a specified interval. The specified interval may optionally follow a pre-determined lag time.

As used herein, a "gelling agent" refers to a compound that, when combined with water and heated to a temperature at about the gelation temperature for that compound, the compound becomes water soluble. Upon cooling, a medium containing the gelling agent then becomes relatively more solid than when such medium containing the gelling agent was heated to a temperature at about the gelation temperature. Gelling agents do not include, for example, polyalkylene oxides such as polyethylene oxide or polyalkylene glycols such as polyethylene glycols.

"Water soluble," as used herein in connection with non-polymeric materials, shall mean from sparingly soluble to very soluble, i.e., not more than 100 parts water required to dissolve 1 part of the non-polymeric, water soluble solute. See Remington, "The Science and Practice of Pharmacy," pages 208 - 209 (2000). "Water soluble," as used herein in connection with polymeric materials, shall mean that the polymer swells in water and can be dispersed at the molecular level to form a homogeneous dispersion or colloidal "solution."

"Semipermeable," as used herein shall mean that water can pass through, yet other molecules including salts and the active ingredients described herein are substantially impermeable.

"Impermeable," as used herein shall mean that the composition does not allow for the passage therethrough of either aqueous fluids, or other molecules such as salts or the active ingredients described herein.

"Diffusible" as used herein shall mean that molecules, such as salts and the active ingredients described herein, can pass through, e.g. out of the dosage form, via diffusion when the dosage form containing such molecules is in contact with an appropriate dissolution medium, e.g. gastro-intestinal fluids or in-vitro dissolution media.

"Erodible" as used herein shall mean the composition dissolves via surface erosion when in contact with an appropriate dissolution medium.

A first embodiment of this invention is a polymeric composition suitable for making a component of a dosage form, e.g., the shell of a dosage form or an edible matrix that may contain an active ingredient, and in particular as an osmotic release dosage form, comprising, consisting of, and/or consisting essentially of, based upon the total wet weight of the composition: a) from about 5 percent to about 50 percent, e.g., from about 10 percent to about 40 percent, of a water insoluble film forming polymer; b) from about 0.05 percent to about 15 percent, e.g., from about 0.1 percent to about 5 percent, of a gelling agent; c) from about 40 percent to about 95 percent, e.g., from about 55 percent to about 90 percent, of a multisolvent system; d) from about 0 percent to about 30 percent, e.g., from about 2 percent to about 25 percent of a plasticizer; and e) from about 0 percent to about 10 percent, e.g., from about 0.05 percent to about 5 percent, of a pore former, wherein said multisolvent system is comprised of, based upon the total weight of the multisolvent system, from about 10 percent to about 50 percent of water and from about 50 percent to about 90 percent of a water miscible organic solvent. Such a polymeric composition may be in the form of a flowable material that is suitable for use as a semipermeable shell or shell portion of a dosage form.

In one embodiment wherein the solvents of this polymeric composition have dried, the polymeric composition is comprised of, based upon the total dry weight of polymeric composition, a) from about 40 percent to about 99 percent, e.g., from about 50 percent to about 80 percent, of a water insoluble film forming polymer; b) from about 1 percent to about 30 percent, e.g., from about 2 percent to about 10 percent, of a gelling agent; c) from about 0 percent to about 60 percent, e.g., from about 1 percent to about 40 percent of a plasticizer; and d) from about 0 percent to about 15 percent, e.g., from about 0.1 percent to about 10 percent, of a pore former. Any water insoluble, semipermeable film forming polymer known in the art is suitable for use in the polymeric composition of the present invention. Suitable water insoluble film-forming polymers include, but are not limited to, cellulose esters, cellulose ethers, cellulose ester-ethers, polyvinyl alcohols, polyvinyl acetate, polycaprolactones, polyacrylates, polymethacrylates, and derivatives, copolymers, and combinations thereof.

The cellulosic water insoluble film forming polymers typically have a degree of substitution (D.S.) on the anhydroglucose unit, from greater than 0 up to 3 inclusive. By "degree of substitution" as used herein it is meant the average number of hydroxyl groups originally present on the anhydroglucose unit comprising the cellulose polymer that are replaced by a substituting group. Representative materials include those selected from the group consisting of cellulose acetate, cellulose diacetate, cellulose triacetate, and derivatives, copolymers, and combinations thereof.

Exemplary water insoluble film forming polymers include cellulose acetate having a D.S. up to 1 and an acetyl content up to 21%; cellulose acetate having an acetyl content of 32 to 39.8%; cellulose acetate having a D.S. of 1 to 2 and an acetyl content of 21 to 35%; cellulose acetate having a D.S. of 2 to 3 and an acetyl content of 35 to 44.8%, cellulose propionate having a D.S. of 1.8 and a propyl content of 39.2 to 45% and a hydroxyl content of 2.8 to 5.4%; cellulose acetate butyrate having a D.S. of 1.8, an acetyl content of 13 to 15% and a butyryl content of 34 to 39%; cellulose acetate butyrate having an acetyl content of 2 to 29%, a butyryl content of 17 to 53% and a hydroxyl content of 0.5 to 4.7%; cellulose triacylates having a D.S. of 2.9 to 3, such as, e.g., cellulose trivalerate, cellulose trilaurate, cellulose tripalmitate, cellulose trisuccinate, and cellulose trioctanoate; cellulose diacylates having a D.S. of 2.2 to 2.6, such as cellulose disuccinate, cellulose dipalmitate, cellulose dioctanoate, and cellulose dipentanoate; co-esters of cellulose, such as cellulose acetate butyrate and cellulose acetate propionate; and derivatives, copolymers, and combinations thereof.

Additional water insoluble film forming polymers include ethyl cellulose of various degree of etherification with ethoxy content of from about 40 to 55%; acetaldehyde demethylcellulose acetate; cellulose acetate ethyl carbamate; cellulose acetate methyl carbamate; cellulose acetate diethyl aminoacetate; semipermeable polyamides; semipermeable polyurethanes; semipermeable sulfonated polystyrenes; semipermeable cross-linked selective polymers formed by the coprecipitation of a polyanion and a polycation as disclosed in U.S. Pat. Nos. 3,173,876; 3,276,586; 4,541,005; 3,541,006, and 3,546,142,; semipermeable polymers as disclosed in U.S. Pat. No. 3,133,132; semipermeable lightly cross-linked poly(-sodium styrene sulfonate); semipermeable cross-linked poly(vinylbenzyltrimethyl ammonium chloride); semipermeable polymers exhibiting a fluid permeability of $2.5 \times 10^{-8}$ to $2.5 \times 10^{-4} (cm^2/hr\ atm)$ expressed per atmosphere of hydrostatic or osmotic pressure difference across the semipermeable wall as set forth in U.S. Pat. Nos. 3,845,770; 3,916,899; and 4,160,020,, and derivatives, copolymers, and combinations thereof.

Suitable gelling agents include but are not limited to hydrocolloids (also referred to herein as gelling polymers), gelling starches, and derivatives, copolymers and mixtures thereof.

Examples of suitable hydrocolloid gelling agents include, but are not limited to alginates, agar, guar gum, locust bean, carrageenan, tara, gum arabic, tragacanth, pectin, xanthan, gellan, maltodextrin, galactomannan, pusstulan, laminarin, scleroglucan, gum arabic, inulin, pectin, whelan, rhamsan, zooglan, methylan, chitin, cyclodextrin, chitosan, and mixtures thereof.

Examples of suitable gelling starches include acid hydrolyzed starches, and derivatives and mixtures thereof.

In one embodiment of the invention, the gelling agent comprises gelatin as a gelling polymer. Gelatin is a natural, thermogelling polymer. It is a tasteless and colorless mixture of derived proteins of the albuminous class which is ordinarily soluble in warm water. Two types of gelatin - Type A and Type B - are commonly used. Type A gelatin is a derivative of acid-treated raw materials. Type B gelatin is a derivative of alkali-treated raw materials. The moisture content of gelatin, as well as its Bloom strength, composition and original gelatin processing conditions, determine its transition temperature between liquid and solid. Bloom is a standard measure of the strength of a gelatin gel, and is roughly correlated with molecular weight. Bloom is defined as the weight in grams required to move a half-inch diameter plastic plunger 4 mm into a 6.67% gelatin gel that has been held at 10°C for 17 hours. In one embodiment, the flowable material is an aqueous solution comprising 20% 275 Bloom pork skin gelatin, 20% 250 Bloom Bone Gelatin, and approximately 60% water.

Additional suitable thickening hydrocolloids include low-moisture polymer solutions such as mixtures of gelatin and other hydrocolloids at water contents up to about 30%, such as for example those used to make "gummi" confection forms.

Suitable xanthan gum gelling agents include those available from C.P. Kelco Company under the tradenames KELTROL 1000, XANTROL 180, or K9B310.

"Acid-hydrolyzed starch," as used herein, is one type of modified starch that results from treating a starch suspension with dilute acid at a temperature below the gelatinization point of the starch. During the acid hydrolysis, the granular form of the starch is maintained in the starch suspension, and the hydrolysis reaction is ended by neutralization, filtration and drying once the desired degree of hydrolysis is reached. As a result, the average molecular size of the starch polymers is reduced. Acid-hydrolyzed starches (also known as "thin boiling starches") tend to have a much lower hot viscosity than the same native starch as well as a strong tendency to gel when cooled.

"Gelling starches," as used herein, include those starches that, when combined with water and heated to a temperature sufficient to form a solution, thereafter form a gel upon cooling to a temperature below the gelation point of the starch. Examples of gelling starches include, but are not limited to, acid hydrolyzed starches such as that available from Grain Processing Corporation under the tradename, " PURE-SET B950;" hydroxypropyl distarch phosphate such as that available from Grain Processing Corporation under the tradename, "PURE-GEL B990," and mixtures thereof.

The multisolvent system is comprised of at least two solvents that are miscible, i.e., are homogeneously dispersable at the molecular level, and when combined together, are capable of dissolving both the water insoluble film forming polymer and the gelling agent. Suitable solvents for use as components of the flowable wet polymeric composition for making the shell, or a portion thereof, by molding include the combination of: a) water; and b) at least one polar organic solvent such as methanol, ethanol, isopropanol, and/or acetone.

In embodiments wherein increased film permeability and flexibility are of concern, optional plasticizers may be added to the polymeric composition. Suitable plasticizers for making the shell, or a portion thereof, by molding include, but are not limited to polyethylene glycol; propylene glycol; glycerin; sorbitol; triethyl citrate; tribuyl citrate; dibutyl sebecate; vegetable oils such as castor oil, grape oil, olive oil, and sesame oil; surfactants such as polysorbates, sodium lauryl sulfates, and dioctyl-sodium sulfosuccinates; mono acetate of glycerol; diacetate of glycerol; triacetate of glycerol; natural gums; triacetin; acetyltributyl citrate; diethyloxalate; diethylmalate; diethyl fumarate; diethylmalonate; dioctylphthalate; dibutylsuccinate; glycerol tributyrate; hydrogenated castor oil; fatty acids such as lauric acid; glycerides such as mono-, di-, and/or triglycerides, which may be substituted with the same or different fatty acids groups such as , for example, stearic, palmitic, and oleic and the like; and/or mixtures thereof. In one embodiment, the plasticizer is triethyl citrate. In certain embodiments, the polymeric composition for the shell is substantially free of plasticizers, i.e. contains less than about 1%, i.e., e.g., than about 0.01 % of plasticizers.

Pore formers may also optionally be added to the polymeric composition. Suitable pore formers include water-soluble organic and inorganic materials. Examples of suitable water-soluble organic materials include water soluble polymers including water soluble cellulose derivatives such as hydroxypropylmethylcellulose, and hydroxypropylcellulose; water soluble carbohydrates such as sugars, and starches; water soluble polymers such as polyvinylpyrrolidone and polyethylene glycol; and insoluble swelling polymers such as microcrystalline cellulose. Examples of suitable water soluble inorganic materials include salts such as sodium chloride and potassium chloride and the like and/or mixtures thereof.

Suitable active ingredients for use in this invention include for example pharmaceuticals, minerals, vitamins and other nutraceuticals, oral care agents, flavorants and mixtures thereof. Suitable pharmaceuticals include analgesics, anti-inflammatory agents, antiarthritics, anesthetics, antihistamines, antitussives, antibiotics, anti-infective agents, antivirals, anticoagulants, antidepressants, antidiabetic agents, antiemetics, antiflatulents, antifungals, antispasmodics, appetite suppressants, bronchodilators, cardiovascular agents, central nervous system agents, central nervous system stimulants, decongestants, oral contraceptives, diuretics, expectorants, gastrointestinal agents, migraine preparations, motion sickness products, mucolytics, muscle relaxants, osteoporosis preparations, polydimethylsiloxanes, respiratory agents, sleep-aids, urinary tract agents and mixtures thereof.

Suitable oral care agents include breath fresheners, tooth whiteners, antimicrobial agents, tooth mineralizers, tooth decay inhibitors, topical anesthetics, mucoprotectants, and the like.

Suitable flavorants include menthol, peppermint, mint flavors, fruit flavors, chocolate, vanilla, bubblegum flavors, coffee flavors, liqueur flavors and combinations and the like.

Examples of suitable gastrointestinal agents include antacids such as calcium carbonate, magnesium hydroxide, magnesium oxide, magnesium carbonate, aluminum hydroxide, sodium bicarbonate, dihydroxyaluminum sodium carbonate; stimulant laxatives, such as bisacodyl, cascara sagrada, danthron, senna, phenolphthalein, aloe, castor oil, ricinoleic acid, and dehydrocholic acid, and mixtures thereof; H2 receptor antagonists, such as famotadine, ranitidine, cimetadine, nizatidine; proton pump inhibitors such as omeprazole or lansoprazole; gastrointestinal cytoprotectives, such as sucraflate and misoprostol; gastrointestinal prokinetics, such as prucalopride, antibiotics for H. pylori, such as clarithromycin, amoxicillin, tetracycline, and metronidazole; antidiarrheals, such as diphenoxylate and loperamide; glycopyrrolate; antiemetics, such as ondansetron, analgesics, such as mesalamine.

In one embodiment of the invention, the active ingredient may be selected from bisacodyl, famotadine, ranitidine, cimetidine, prucalopride, diphenoxylate, loperamide, lactase, mesalamine, bismuth, antacids, and pharmaceutically acceptable salts, esters, isomers, and mixtures thereof.

In another embodiment, the active ingredient is selected from analgesics, antiinflammatories, and antipyretics, e.g. non-steroidal anti-inflammatory drugs (NSAIDs), including propionic acid derivatives, e.g. ibuprofen, naproxen, ketoprofen and the like; acetic acid derivatives, e.g. indomethacin, diclofenac, sulindac, tolmetin, and the like; fenamic acid derivatives, e.g. mefenamic acid, meclofenamic acid, flufenamic acid, and the like; biphenylcarbodylic acid derivatives, e.g. diflunisal, flufenisal, and the like; and oxicams, e.g. piroxicam, sudoxicam, isoxicam, meloxicam, and the like. In one particular embodiment, the active ingredient is selected from propionic acid derivative NSAID, e.g. ibuprofen, naproxen, flurbiprofen, fenbufen, fenoprofen, indoprofen, ketoprofen, fluprofen, pirprofen, carprofen, oxaprozin, pranoprofen, suprofen, and pharmaceutically acceptable salts, derivatives, and combinations thereof. In another particular embodiment of the invention, the active ingredient may be selected from acetaminophen, acetyl salicylic acid, ibuprofen, naproxen, ketoprofen, flurbiprofen, diclofenac, cyclobenzaprine, meloxicam, rofecoxib, celecoxib, and pharmaceutically acceptable salts, esters, isomers, and mixtures thereof.

In another embodiment of the invention, the active ingredient may be selected from pseudoephedrine, phenylpropanolamine, chlorpheniramine, dextromethorphan, diphenhydramine, astemizole, terfenadine, fexofenadine, loratadine, desloratadine, cetirizine, mixtures thereof and pharmaceutically acceptable salts, esters, isomers, and mixtures thereof.

Examples of suitable polydimethylsiloxanes, which include, but are not limited to dimethicone and simethicone, are those disclosed in United States Patent Nos. 4,906,478, 5,275,822, and 6,103,260. As used herein, the term "simethicone" refers to the broader class of polydimethylsiloxanes, including but not limited to simethicone and dimethicone.

The polymeric composition of the present invention may be made by: (a) combining the water with all other organic solvents to form a multicomponent solvent mixture under ambient conditions; (b) adding the water insoluble film forming polymer(s) thereto with stirring under ambient conditions; (c) increasing the temperature of the mixture of step (b) to a temperature that is greater than the gelling temperature of the desired gelling agent(s), then adding the gelling agent(s) thereto with stirring until the mixture is homogenous while maintaining a constant temperature. In embodiments wherein plasticizers and/or pore formers are included in the composition, these ingredients are typically added to the mixture at any time after step (a). Alternatively, the polymeric composition of the present invention may be made by: (a) combining the organic solvents and the desired film former(s) to form a first pre-mixture in a first container under ambient conditions; (b) combining the water and the gelling agent(s) in a second container at a temperature above the gelling temperature of the gelling agent(s) to form a second pre-mixture; (c) combining the first pre-mixture and the second pre-mixture at a temperature above the gelling temperature of the gelling agent(s) until the resulting mixture is homogeneous.

Another embodiment of the present invention is depicted in Figure 1A, which is a cross-sectional view of a dosage form 2 which comprises a core 4 and a shell 5. In other embodiments of this invention, shell 5 may reside upon a portion of core 4 without completely surrounding the core 4. In these embodiments of the invention, shell 5 is semipermeable to a liquid medium, and at least a portion of such shell is comprised of the polymeric composition described above. Core 4 contains at least one active ingredient. In this embodiment of the invention, shell 5 contains passageway 12 that extends from the outer surface of shell 5 to at least the outer surface of core 4, as shown. In other embodiments of this invention, a plurality of passageways may be employed. Accordingly, upon contacting of dosage form 2 with a liquid medium, the liquid medium permeates shell 5, reaches core 4 (where active ingredient, and optionally osmagent or osmopolymer, are contained), and liquid medium containing active ingredient is osmotically "pumped" through passageway 12 and out of dosage form 2 into the surrounding liquid medium.

Another embodiment of this invention is depicted in Figure 1 B, which is a cross-sectional view of a dosage form 22 which comprises a core 24, a first shell portion 25 and a second shell portion 26, which in this embodiment surround core 24. In other embodiments of this invention, first shell portion 25 or second shell portion 26 may reside upon a portion of core 24 without completely surrounding core 24. First shell portion 25 is semipermeable to a liquid medium is comprised of the polymeric composition described above, and second shell portion 26 is compositionally different than first shell portion 25. In the embodiment depicted in Figure 1B, second shell portion 26 is diffusible. Core 24 contains at least one active ingredient. Accordingly, upon contacting of dosage form 22 with a liquid medium, the liquid medium permeates first shell portion 25, reaches core 24 (where active ingredient is contained), and liquid medium containing active ingredient is osmotically "pumped" through diffusible second shell portion 26 and out of dosage form 22 into the surrounding liquid medium. In other embodiments, second shell portion 26 may be erodible, thereby permitting active ingredient to be released from the core 24 as second shell portion 26 erodes to expose core 24 to the liquid medium.

Another embodiment of this invention is depicted in Figure 2, which is a cross-sectional side view of a dosage form 202 that comprises a core 204 and a shell 206 having a first shell portion 208 which is semipermeable to the liquid medium and is comprised of the polymeric composition set forth above, and a second shell portion 210 which is compositionally different than first shell portion 208. For example, second shell portion 210 may be diffusible, impermeable or erodible. Core 204 contains at least one active ingredient. In this embodiment of the invention, first shell portion 208 contains passageway 212 which extends from the outer surface of first shell portion 208 to the outer surface of core 204, as shown. In other embodiments of this invention, first shell portion 208 may contain a plurality of passageways. In this embodiment, first shell portion 208 is semipermeable, and second shell portion 210 is impermeable. Accordingly, upon contacting of dosage form 202 with a liquid medium, the liquid medium permeates first shell portion 208, reaches core 204 (where active ingredient, and optionally osmagent or osmopolymer are contained), and liquid medium containing active ingredient is osmotically "pumped" through passageway 212 and out of dosage form 202 into the surrounding liquid medium. In other embodiments of this invention (not shown in Figure 2), second shell portion 210 may contain at least one passageway which extends from the outer surface of second shell portion 210 to the outer surface of core 204, or both first shell portion 208 and second shell portion 210 may each contain at least one passageway which extends from the outer surface of first shell portion 208 and second shell portion 210, respectively, to the outer surface of core 204.

Another embodiment of this invention is depicted in Figure 3, which is a cross-sectional side view of a dosage form 302 which comprises a core 304 having first core portion 303 and second core portion 305, and shell 306 having a plurality of passageways 312 therein which extend from the outer surface of shell 306 to the outer surfaces of first and second core portions 303 and 305. In other embodiments of this invention, shell 306 may have a single passageway. Core 304 contains at least one active ingredient. At least a portion of shell 306 is semipermeable to a liquid medium and is comprised of the polymeric composition described above, and at least about 30% of the cross-sectional area of the semipermeable portion of the shell is non-striated. Upon contacting of dosage form 302 with a liquid medium, the liquid medium permeates shell 306, reaches first and second core portions 303 and 305, respectively, (where active ingredient or ingredients, and optionally osmagent or osmopolymer are contained), and liquid medium containing active ingredient or ingredients is osmotically "pumped" through passageways 312 and out of dosage form 302 into the surrounding liquid medium. In one embodiment, first core portion 303 contains a first active ingredient and second core portion 305 contains a second active ingredient which may be the same or different than the first active ingredient. In another embodiment, first and second core portions 303 and 305 each contain a different dose or concentration of first and second active ingredients, which may be the same or different active ingredients. In another embodiment, passageways 312 in shell 306 may expose different surface areas of the underlying core portions 303 and 305, respectively, thereby permitting either different release rates for the first and second active ingredients, or the same release rate for the first and second active ingredients, if the first and second active ingredients have different solubilities.

Another embodiment of this invention is depicted in Figure 4, which is a cross-sectional side view of a dosage form 402 which comprises a core 404 having first core portion 403, second core portion 405, and third core portion 407, and shell 406 comprising first and second shell portions 408 and 410, respectively. A plurality of passageways 412 extend from the outer surface of shell 406 to the outer surfaces of first and second core portions 403 and 405. In other embodiments of this invention, shell 406 may have a single passageway located in either first shell portion 408 or second shell portion 410. In this embodiment, first shell portion 408 and second shell portion 410 are each semipermeable to a liquid medium, and at least a portion of the first shell portion 408 or the second shell portion 410 is comprised of the polymeric composition described above. Second shell portion 410 may be compositionally different than first shell portion 408. For example, in other embodiments first shell portion 408 may be semipermeable and comprised of the polymeric composition described above, and second shell portion 410 may be diffusible, impermeable or erodible. Core 404 contains at least one active ingredient. In the embodiment depicted in Figure 4, first core portion 403 contains a first active ingredient, second core portion 405 contains a second active ingredient which may be the same or different than the first active ingredient, and third core portion 407 contains an osmopolymer which provides osmotic pressure upon contact with the liquid medium to push the active ingredients through the passageways 412 to the surface of shell 406. In the embodiment depicted in Figure 4, upon contacting of dosage form 402 with a liquid medium, the liquid medium permeates the first and second shell portions 408 and 410, reaches first and second core portions 403 and 405, respectively (where active ingredient or ingredients are contained), as well as third core portion 407 containing the osmopolymer (which swells and compresses against first and second core portions 403 and 405), and liquid medium containing active ingredient is osmotically "pumped" through passageways 412 and out of dosage form 402 into the surrounding liquid medium. In addition, passageways 412 in first and second shell portions 408 and 410 may expose different surface areas of the underlying core portions 403 and 405, respectively, thereby permitting either different release rates for the first and second active ingredients, or the same release rate for the first and second active ingredients, if the first and second active ingredients have different solubilities.

Another embodiment of this invention is depicted in Figure 5, which is a cross-sectional side view of a dosage form 502 that comprises a core 504 and a shell 506 having a first shell portion 508 which is semipermeable to the liquid medium and is comprised of the polymeric composition described above, and a second shell portion 510 which may be compositionally different than first shell portion 508. For example, second shell portion 510 may be semipermeable, diffusible, impermeable or erodible, although in this embodiment second shell portion 510 is semipermeable. Core 504 contains at least one active ingredient. As depicted in Figure 5, cavity 501 extends through core 504, first shell portion 508 and second shell portion 510. The interior surface 513 of core 504 is defined by cavity 501, and neither first shell portion 508 nor second shell portion 510 substantially extend upon interior surface 513, thereby permitting active ingredient contained within core 504 to be released only through interior surface 513. The diameter of the continuous cavity is typically in the range of about 15 to about 90 percent of the thickness of the dosage form, and the diameter of the continuous cavity is typically in the range of about 5 to about 30 percent of the core diameter. The length of the continuous cavity is typically about the same as the thickness of the dosage form, and the length of the continuous cavity is typically about 25 to about 40 percent of the diameter of the dosage form. Upon contacting of dosage form 502 with a liquid medium, the liquid medium permeates interior surface 513, as well as first and second shell portions 508 and 510, reaches core 504 (where active ingredient is contained), and liquid medium containing active ingredient passes through interior surface 513 into central cavity 501 and out of dosage form 502 into the surrounding liquid medium. In this embodiment, the predominant mechanism for drug release is erosion.

Another embodiment of this invention is depicted in Figure 6, which is a cross-sectional side view of a dosage form 602 which comprises a core 604 having first core portion 603, second core portion 605, and third core portion 607, and a shell 606 having a first shell portion 608 which is semipermeable to the liquid medium and is comprised of the polymeric composition described above, and a second shell portion 610 which is compositionally different than first shell portion 608. For example, second shell portion 610 may be semipermeable, diffusible, impermeable or erodible, although in this embodiment second shell portion 610 is semipermeable. Core 604 contains at least one active ingredient. As depicted in Figure 6, cavity 601 extends through core 604, first shell portion 608 and second shell portion 610. The interior surface 613 of core 604 is defined by cavity 601, and neither first shell portion 608 nor second shell portion 610 substantially extend upon interior surface 613, thereby permitting active ingredient contained within core 604 to be released only through interior surface 613. In one embodiment, first core portion 603 contains a first active ingredient, second core portion 605 contains a second active ingredient which may be the same or different than the first active ingredient, and third core portion 607 contains a osmopolymer which provides osmotic pressure upon contact with the liquid medium to push the active ingredients through the interior surface 613 and into the liquid medium. The diameter of the continuous cavity is typically in the range of about 15 to about 90 percent of the thickness of the dosage form, and the diameter of the continuous cavity is typically in the range of about 5 to about 30 percent of the core diameter. The length of the continuous cavity is typically about the same as the thickness of the dosage form, and the length of the continuous cavity is typically about 25 to about 40 percent of the diameter of the dosage form. Upon contacting of dosage form 602 with the liquid medium, the liquid medium permeates interior surface 613, as well as the first and second shell portions 608 and 610, respectively, reaches first core portion and second core portions 603 and 605, respectively, (where active ingredient or ingredients are contained) as well as third core portion 607 containing the osmopolymer (which swells and compresses against first core portion 603 and second core portion 605), and liquid medium containing the active ingredient or ingredients passes through interior surface 613 into central cavity 601 and out of dosage form 602 into the surrounding liquid medium. In this embodiment, the predominant mechanism for drug release is erosion.

In embodiments of this invention in which the shell comprises at least two shell portions, the inner surface of each shell portion must be substantially in contact with the outer surface of the core, as depicted, for example, in Figures 1 B, 2, 4, 5 and 6. Accordingly, in such embodiments, the inner surface of any shell portion does not reside upon the outer surface of any other shell portion.

The active ingredient is present in the dosage form in a therapeutically effective amount, which is an amount that produces the desired therapeutic response upon oral administration and can be readily determined by one skilled in the art. In determining such amounts, the particular active ingredient being administered, the bioavailability characteristics of the active ingredient, the dosing regimen, the age and weight of the patient, and other factors must be considered, as known in the art. Typically, the dosage form comprises about 2 to about 75 weight percent, for example, the dosage form may comprise about 5 to about 50 weight percent, say about 7 to about 25 weight percent of a combination of one or more active ingredients. In one embodiment, the core comprises a total of at least about 25 weight percent, e.g. about 25 to about 75 weight percent (based on the weight of the core) of one or more active ingredients.

The active ingredient may be present in the dosage form in any form. For example, the active ingredient may be dispersed at the molecular level, e.g. melted or dissolved, within the dosage form, or may be in the form of particles, which in turn may be coated or uncoated. If the active ingredient is in form of particles, the particles (whether coated or uncoated) typically have an average particle size of about 1 micron to about 2000 microns. In one embodiment, such particles are crystals having an average particle size of about 1 micron to about 300 microns. In another embodiment, the particles are granules or pellets having an average particle size of about 50 microns to about 2000 microns, e.g. about 50 microns to about 1000 microns, or about 100 microns to about 800 microns.

In embodiments where an active ingredient is contained within the core, at least a portion of the active ingredient may be optionally coated with a release-modifying coating, as known in the art. This advantageously provides an additional tool for modifying the release profile of active ingredient from the dosage form. For example, the core may contain coated particles of one or more active ingredients, in which the particle coating confers a release modifying function, as is well known in the art. Examples of suitable release modifying coatings for particles are described in U.S. Patent Nos. 4,173,626; 4,863,742; 4,980,170; 4,984,240; 5,286,497; 5,912,013; 6,270,805; and 6,322,819. Commercially available modified release coated active particles may also be employed. Accordingly, all or a portion of one or more active ingredients in the core may be coated with a release-modifying material.

In embodiments in which it is desired for the active ingredient to be absorbed into the systemic circulation of an animal, the active ingredient or ingredients are typically capable of dissolution upon contact with a fluid such as water, gastric fluid, intestinal fluid or the like. In one embodiment, the dissolution characteristics of one or more active ingredients may be modified: e.g. controlled, sustained, extended, retarded, prolonged, delayed and the like. In one embodiment in which one or more active ingredients are released in a modified manner, the modified release active ingredient or ingredients are contained in the core. In one particular such embodiment, the dosage form releases one or more active ingredients contained in the core at a substantially constant rate over a specified time interval.

In one embodiment, the dissolution characteristics of at least one active ingredient meets USP specifications for immediate release tablets containing the active ingredient. For example, for acetaminophen tablets, USP 24 specifies that in pH 5.8 phosphate buffer, using USP apparatus 2 (paddles) at 50 rpm, at least 80% of the acetaminophen contained in the dosage form is released therefrom within 30 minutes after dosing, and for ibuprofen tablets, USP 24 specifies that in pH 7.2 phosphate buffer, using USP apparatus 2 (paddles) at 50 rpm, at least 80% of the ibuprofen contained in the dosage form is released therefrom within 60 minutes after dosing. See USP 24, 2000 Version, 19 - 20 and 856 (1999).

In embodiments in which at least one active ingredient is released immediately, the immediately released active ingredient is typically contained in the shell or on the surface of the shell, e.g. in a further coating surrounding at least a portion of the shell.

In certain embodiments, the core or core portions function as an eroding matrix from which dispersed active ingredient is liberated by the dissolution of successive layers of the matrix surface. In these embodiments, the rate of active ingredient release from the core or core portion will depend on the dissolution rate of the matrix material. Particularly useful eroding matrix materials for providing surface erosion include those which first absorb liquid, then swell and/or gel prior to dissolving. In certain such embodiments, the eroding matrix core or core portion comprises, based upon the total dry weight of the core or core portion, from about 5 percent to about 50 percent of a release-modifying compressible or moldable excipients selected from swellable erodible hydrophilic materials, pH-dependent polymers, insoluble edible materials, and combinations thereof. In one embodiment, suitable release-modifying excipients for making the core, or the shell, or a portion thereof, by molding include hydroxypropylcellulose, hydroxypropylmethylcellulose, polyethylene oxide, ammonio methacrylate copolymer type B, and shellac, and combinations thereof.

Suitable swellable erodible hydrophilic materials for use as release-modifying excipients for making the core, or a portion thereof, by compression include: water swellable cellulose derivatives, polyalkylene glycols, polyalkylene oxides, acrylic polymers, hydrocolloids, gelling starches, and swelling cross-linked polymers, and derivatives, copolymers, and combinations thereof.

Examples of suitable water swellable cellulose derivatives include sodium carboxymethylcellulose, cross-linked hydroxypropylcellulose, hydroxypropyl cellulose (HPC), hydroxypropylmethylcellulose (HPMC), hydroxyisopropylcellulose, hydroxybutylcellulose, hydroxyphenylcellulose, hydroxyethylcellulose (HEC), hydroxypentylcellulose, hydroxypropylethylcellulose, hydroxypropylbutylcellulose, and hydroxypropylethylcellulose.

Examples of suitable polyalkylene glycols include polyethylene glycol. Examples of suitable thermoplastic polyalkylene oxides include poly (ethylene oxide).

Examples of suitable acrylic polymers include potassium methacrylatedivinylbenzene copolymer, polymethylmethacrylate, CARBOPOL (high-molecular weight cross-linked acrylic acid homopolymers and copolymers), and the like.

Examples of suitable hydrocolloids include those set forth above.

Examples of suitable clays include smectites such as bentonite, kaolin, and laponite; magnesium trisilicate, magnesium aluminum silicate, and the like, and derivatives and mixtures thereof.

Examples of suitable gelling starches include acid hydrolyzed starches, swelling starches such as sodium starch glycolate, and derivatives thereof.

Examples of suitable swelling cross-linked polymers include cross-linked polyvinyl pyrrolidone, cross-linked agar, and cross-linked carboxymethylcellose sodium.

Suitable insoluble edible materials for use as release-modifying excipients for making the core, or a portion thereof, by compression include water-insoluble polymers, and low-melting hydrophobic materials.

Examples of suitable water-insoluble polymers include ethylcellulose, polyvinyl alcohols, polyvinyl acetate, polycaprolactones, cellulose acetate and its derivatives, acrylates, methacrylates, acrylic acid copolymers; and the like and derivatives, copolymers, and combinations thereof.

Suitable low-melting hydrophobic materials include fats, fatty acid esters, phospholipids, and waxes. Examples of suitable fats include hydrogenated vegetable oils such as for example cocoa butter, hydrogenated palm kernel oil, hydrogenated cottonseed oil, hydrogenated sunflower oil, and hydrogenated soybean oil; and free fatty acids and their salts. Examples of suitable fatty acid esters include sucrose fatty acid esters, mono, di, and triglycerides, glyceryl behenate, glyceryl palmitostearate, glyceryl monostearate, glyceryl tristearate, glyceryl trilaurylate, glyceryl myristate, GLYCOWAX-932, lauroyl macrogol-32 glycerides, and stearoyl macrogol-32 glycerides. Examples of suitable phospholipids include phosphotidyl choline, phosphotidyl serene, phosphotidyl enositol, and phosphotidic acid. Examples of suitable waxes include carnauba wax, spermaceti wax, beeswax, candelilla wax, shellac wax, microcrystalline wax, and paraffin wax; fat-containing mixtures such as chocolate; and the like.

Suitable pH-dependent polymers for use as release-modifying excipients for making the core, or a portion thereof, by compression include enteric cellulose derivatives, for example hydroxypropyl methylcellulose phthalate, hydroxypropyl methylcellulose acetate succinate, cellulose acetate phthalate; natural resins such as shellac and zein; enteric acetate derivatives such as for example polyvinylacetate phthalate, cellulose acetate phthalate, acetaldehyde dimethylcellulose acetate; and enteric acrylate derivatives such as for example polymethacrylate-based polymers such as poly(methacrylic acid, methyl methacrylate) 1:2, which is commercially available from Rohm Pharma GmbH under the tradename, "EUDRAGIT S," and poly(methacrylic acid, methyl methacrylate) 1:1, which is commercially available from Rohm Pharma GmbH under the tradename, "EUDRAGIT L,"and the like, and derivatives, salts, copolymers, and combinations thereof.

In certain other embodiments, the core or core portions function as a diffusional matrix. In these embodiments, the core portion comprises active ingredient, distributed throughout an insoluble porous matrix, which contains pores or channels through which fluids can enter the core or core portion, and the active ingredient must diffuse to be released from the dosage form. In these embodiments, the rate of active ingredient release from the core or core portion will depend upon the area (A) of the matrix, the diffusion coefficient (D), the porosity (E) and tortuosity (T) of the matrix; the drug solubility (Cs) in the dissolution medium; and the drug concentration (Cp) in the dosage form. In embodiments in which a core or core portion functions as a diffusional matrix, the release of the active ingredient from the core or core portion may be described as controlled, prolonged, sustained, or extended. In these embodiments, the contribution to active ingredient dissolution from the subject core portion may follow zero-order, first-order, or square-root of time kinetics. In certain such embodiments, the diffusional matrix core or core portion may comprise a pore former such as those set forth above.

In embodiments in which the core or core portion functions to modify release of an active ingredient contained therein, the release of active ingredient may be further modified by the function of the surrounding shell or shell portion, as described above. In such embodiments, the release of the active ingredient from the dosage form will be governed by the sum of all the contributions acting upon it, e.g. from the relevant core or core portion and shell or shell portions, and may be described as controlled, prolonged, sustained, extended, delayed, or pulsatile. In these embodiments, the dissolution of active ingredient from the dosage form may follow zero-order, first-order, or square-root of time kinetics.

In embodiments in which the core comprises multiple portions, the portions may comprise different materials, or be prepared by different methods, or both. In one particular embodiment a first core portion may be prepared by compression, and a second core portion may be prepared by molding.

In certain embodiments, the core comprises multiple portions, which comprise different active ingredients or have different release-modifying properties, or both; and the shell comprises a corresponding number of multiple portions, which each cover a specific core portion in order to modify or further modify the release of one or more active ingredients contained within the respective core portion. For such embodiments, it is critical to have a manufacturing process which is capable of maintaining the orientation of the core prior to and during the application of the shell or each shell portion thereon. Advantageously, the orientation of the components of the dosage forms of the present invention can be precisely controlled, when manufactured using the thermal cycle or thermal setting apparatus and described below.

In one such embodiment, the dosage form comprises a core comprising a first core portion and a second core portion which are compositionally different, wherein at least one of the first or second core portions comprises an active ingredient; and a shell which surrounds the core and comprises a first shell portion and a second shell portion which are compositionally different, wherein at least one of the first or second shell portions confers a modification to the release of an active ingredient contained in the underlying core portion.

The core or core portion of the present invention may be prepared by any suitable method, including for example compression and molding, and depending on the method by which it is made, typically comprises active ingredient and a variety of excipients (inactive ingredients which may be useful for conferring desired physical properties to the core).

In embodiments in which the core, or a portion thereof, is made by compression, suitable excipients include fillers, binders, disintegrants, lubricants, glidants, and the like, as known in the art. In embodiments in which the core is made by compression and additionally confers modified release of an active ingredient contained therein, the core may further comprises a release-modifying compressible excipient as disclosed above.

Suitable filters for use in making the core, or a portion thereof, by compression include water-soluble compressible carbohydrates such as sugars, which include dextrose, sucrose, maltose, and lactose, sugar-alcohols, which include mannitol, sorbitol, maltitol, xylitol, starch hydrolysates, which include dextrins, and maltodextrins, and the like, water insoluble plastically deforming materials such as microcrystalline cellulose or other cellulosic derivatives, water-insoluble brittle fracture materials such as dicalcium phosphate, tricalcium phosphate and the like and mixtures thereof.

Suitable binders for making the core, or a portion thereof, by compression include dry binders such as polyvinyl pyrrolidone, hydroxypropylmethylcellulose, and the like; wet binders such as water-soluble polymers, including hydrocolloids such as acacia, alginates, agar, guar gum, locust bean, carrageenan, carboxymethylcellulose, tara, gum arabic, tragacanth, pectin, xanthan, gellan, gelatin, maltodextrin, galactomannan, pusstulan, laminarin, scleroglucan, inulin, whelan, rhamsan, zooglan, methylan, chitin, cyclodextrin, chitosan, polyvinyl pyrrolidone, cellulosics, sucrose, starches, and the like; and derivatives and mixtures thereof.

Suitable disintegrants for making the core, or a portion thereof, by compression, include sodium starch glycolate, cross-linked polyvinylpyrrolidone, cross-linked carboxymethylcellulose, starches, microcrystalline cellulose, and the like.

Suitable lubricants for making the core, or a portion thereof, by compression include long chain fatty acids and their salts, such as magnesium stearate and stearic acid, talc, glycerides and waxes.

Suitable glidants for making the core, or a portion thereof, by compression, include colloidal silicon dioxide, and the like.

Other suitable pharmaceutically acceptable adjuvants for making the core, or a portion thereof, by compression include, preservatives; high intensity sweeteners such as aspartame, acesulfame potassium, sucralose, and saccharin; flavorants; colorants; antioxidants; surfactants; wetting agents; and the like and mixtures thereof.

In one embodiment, the core or a portion thereof comprises at least one osmagent, which is also known as an osmotically effective solute or an osmotically effective compound that can be blended homogeneously or heterogeneously with the core constituents to form a push member, acting as osmotically effective solutes that are soluble in liquid medium imbibed into the core, and exhibit an osmotic pressure gradient across the semipermeable shell or shell portion against an exterior liquid medium. Osmagents useful in the present invention include, but are not limited to solid compounds selected from the group consisting of lithium chloride, magnesium sulfate, potassium sulfate, magnesium chloride, sodium chloride, sodium sulfate, sorbitol, mannitol, urea, inositol, sucrose, glucose, and mixtures thereof. The osmotic pressure in atmospheres ("ATM") of the osmagents suitable for the invention with be greater than zero ATM, generally from zero ATM up to 500 ATM, or higher. The amount of osmagent blended with the core constituents is, based upon the total dry weight of the core, from about 0 percent to about 65 percent, e.g., from about 0.01 percent to about 40 percent.

In another embodiment, the core or a portion thereof comprises at least one osmopolymer. The osmopolymer, if employed, exhibits fluid absorbing and or fluid imbibing properties. The osmopolymer comprises a hydrophilic polymer that can interact with water and aqueous biological fluids and then swell or expand to an equilibrium state. The osmopolymer exhibits the ability to retain a significant portion of the imbibed or absorbed fluid. Examples of suitable osmopolymers include, but are not limited to poly(hydroxyalkyl methacrylate) having a molecular weight of 20,000 to 5,000,000; poly(vinylpyrrolidone) having a molecular weight of about 10,000 to 360,000; poly(vinylalcohol) having a low acetate content and lightly cross-linked with glyoxal, formaldehyde, or glutaraldehyde and having a degree of polymerization from 2,000 to 30,000; poly(ethylene oxide) having a molecular weight from 10,000 to 7,800,000; acidic carboxy polymers known as carboxypolymethylene or as carboxyvinyl polymers; and mixtures thereof. Examples of suitable acidic carboxypolymers include, but are not limited to a polymer consisting of acrylic acid lightly cross-linked with polyallylsucrose and sold under the trade name, "CARBOPOL;" an acidic carboxy polymer having a molecular weight of 200,000 to 6,000,000, including sodium acidic carboxyvinyl hydrogel and potassium acidic carboxyvinyl hydrogel; a polyacrylamide sold under the tradename, "CYANAMER;" and mixtures and copolymers thereof. Representative osmopolymers, used for the purpose of the present invention, are known to those skilled in the art and described, for example, in Scott & Roff, Handbook of Common Polymers pp. 72 to 81, 98, 281(1971); Ratner & Hoffman, ACS Symposium Series, No. 31, pp. 1 to 36 (1976) (published by the American Chemical Society); and Schacht, Recent Advances in Drug Delivery Systems, 259 to 278 (1984). The amount of osmopolymer blended with the core constituents is, based upon the total dry weight of the core, from about 0 percent to about 90 percent, e.g., from about 20 percent to about 50 percent.

In embodiments in which the core or core portion is prepared by compression, a dry blending (i.e. direct compression), or wet granulation process may be employed. In a dry blending (direct compression) method, the active ingredient or ingredients, together with the excipients, are blended in a suitable blender, then transferred directly to a compression machine for pressing into tablets. In a wet granulation method, the active ingredient or ingredients, appropriate excipients, and a solution or dispersion of a wet binder (e.g. an aqueous cooked starch paste, or solution of polyvinyl pyrrolidone) are mixed and granulated. Alternatively a dry binder may be included among the excipients, and the mixture may be granulated with water or other suitable solvent. Suitable equipment for wet granulation are known in the art, including low shear, e.g. planetary mixers; high shear mixers; and fluid beds, including rotary fluid beds. The resulting granulated material is dried, and optionally dry-blended with further ingredients, e.g. adjuvants and/or excipients such as for example lubricants, colorants, and the like. The final dry blend is then suitable for compression. Methods for direct compression and wet granulation processes are known in the art, and are described in detail in, for example, Lachman, et al., The Theory and Practice of Industrial Pharmacy. Chapter 11 (3rd ed. 1986).

The dry-blended, or wet granulated, powder mixture is typically compacted into tablets using a rotary compression machine as known in the art, such as for example those commercially available from Fette America Inc. (Rockaway, NJ), or Manesty Machines LTD (Liverpool, UK). In a rotary compression machine, a metered volume of powder is filled into a die cavity, which rotates as part of a "die table" from the filling position to a compaction position where the powder is compacted between an upper and a lower punch to an ejection position, where the resulting tablet is pushed from the die cavity by the lower punch and guided to an ejection chute by a stationary "take-off" bar.

In one particular embodiment, the core or core portion may be prepared by the compression methods and apparatus described in United States Patent Publication No. US2003/0072799. Specifically, the core is made using a rotary compression module comprising a fill zone, insertion zone, compression zone, ejection zone, and purge zone in a single apparatus having a double row die construction as shown in Figure 6 of United States Patent Publication No. US2003/0072799. The dies of the compression module are typically filled using the assistance of a vacuum, with filters located in or near each die. The purge zone of the compression module includes an optional powder recovery system to recover excess powder from the filters and return the powder to the dies.

In other embodiments of this invention, the core, or the shell, or a portion thereof, may be prepared by molding. In particular, the core, the shell or a portion of either one may be made by solvent-based molding, or the core or a portion of the shell may be made by solvent-free molding. In such embodiments, the core, or the shell, or a portion thereof, may be made from a flowable material optionally comprising an active ingredient. The flowable material may be any edible material that is flowable at a temperature between about 37°C and 250°C, and that is solid, semi-solid, or can form a gel at a temperature between about - 10°C and about 35°C. When it is in the fluid or flowable state, the flowable material may comprise a dispersed, dissolved, or molten component, and with respect to the shell formulation of a portion of the shell, a solvent such as for example water or organic solvents, or combinations thereof. The solvent may be partially or substantially removed by drying. At least a portion of the shell is comprised of the polymeric composition described above.

In one embodiment, solvent-based or solvent-free molding is performed via thermal setting molding using the method and apparatus described in United States Patent Publication No. US2003/0124183. In this embodiment, a core or a portion of the shell is formed by injecting flowable material into a molding chamber. The flowable material may comprise a thermal setting material at a temperature above its melting point but below the decomposition temperature of any active ingredient contained therein. The flowable material is cooled and solidifies in the molding chamber into a shaped form (i.e., having the shape of the mold).

According to this thermal setting molding method, the flowable material may comprise solid particles suspended in a molten matrix, for example a polymer matrix. The flowable material may be completely molten or in the form of a paste. The flowable material may comprise an active ingredient dissolved in a molten material in the case of solvent-based molding. Alternatively, the flowable material may be made by dissolving a solid in a solvent, which solvent is then evaporated after the molding step in the case of solvent-based molding. At least a portion of the shell is comprised of the polymeric composition described above.

In another embodiment, solvent-based or solvent-free molding is performed by thermal cycle molding using the method and apparatus described in United States Patent Publication No. US2003/0086973A1. Thermal cycle molding is performed by injecting a flowable material into a heated molding chamber. The flowable material may comprise active ingredient and a thermoplastic material at a temperature above the set temperature of the thermoplastic material but below the decomposition temperature of active ingredient. The flowable material is cooled and solidifies in the molding chamber into a shaped form (i.e., having the shape of the mold). At least a portion of the shell is comprised of the polymeric composition described above.

In the thermal cycle molding method and apparatus of United States Patent Publication No. US 2003/0086973A1, a thermal cycle molding module having the general configuration shown in Figure 3 therein is employed. The thermal cycle molding module 200 comprises a rotor 202 around which a plurality of mold units 204 are disposed. The thermal cycle molding module includes a reservoir 206 (see Figure 4 therein) for holding flowable material to make the core. In addition, the thermal cycle molding module is provided with a temperature control system for rapidly heating and cooling the mold units. Figures 55 and 56 therein depict the temperature control system 600.

According to this thermal cycle molding method, the mold units may comprise center mold assemblies 212, upper mold assemblies 214, and lower mold assemblies 210, as shown in Figures 26-28 therein, which mate to form mold cavities having a desired shape, for instance of a core or a shell surrounding a core. As rotor 202 rotates, opposing center and upper mold assemblies or opposing center and lower mold assemblies close. Flowable material, which is heated to a flowable state in reservoir 206, is injected into the resulting mold cavities. The temperature of the flowable material is then decreased, hardening the flowable material. The mold assemblies open and eject the finished product.

In one embodiment of the invention, the shell may be applied to the dosage form using a thermal cycle molding apparatus of the general type shown in Figures 28A-C of United States Patent Publication No. US 2003/0086973A1. The apparatus is comprised of rotatable center mold assemblies 212, lower mold assemblies 210 and upper mold assemblies 214. Cores are continuously fed to the mold assemblies. Shell flowable material, which may be comprised of the polymeric composition described above, is heated to a flowable state in reservoir 206, and then is injected into the mold cavities created by the closed mold assemblies holding the cores. The temperature of the shell flowable material is then decreased, hardening it around the cores. The mold assemblies open and eject the finished dosage forms. Shell coating is performed in two steps, each half of the dosage forms being coated separately as shown in the flow diagram of Figure 28B of United States Patent Publication No. US 2003/0068367 via rotation of the center mold assembly.

One method for making a dosage form having a shell or a shell portion from a wet flowable material, such as the wet polymeric composition described above, is via a solvent-based molding method comprised of: (a) preparing a flowable dispersion of either the polymeric composition described above, or another composition comprised of, for example, film former, gelling agent, optional active ingredient, optional plasticizer, optional release-modifying excipient, and other shell materials in a suitable solvent; (b) injecting the flowable dispersion (which may be heated in a heated feed tank) into a mold cavity (at room temp or below) containing a core such that the flowable dispersion surrounds a first portion of the core within the mold cavity; (c) rapidly changing the temperature of the mold cavity to induce thermal setting of the flowable dispersion around at first portion of the core, i.e., to reduce the temperature to that which is below the gelation temperature of the flowable dispersion; (d) opening the mold cavity and rotating the portion of the mold containing the core to expose a second portion of the core; (e) closing the mold cavity; (f) injecting a heated, flowable dispersion, which may be the same or different from the flowable material used to form the first portion, into the mold cavity such that the flowable dispersion surrounds the second portion of the core within the mold cavity; (g) rapidly changing the temperature of the mold cavity to induce thermal setting of the flowable dispersion surrounding the second portion of the core, i.e., to reduce the temperature to that which is below the gelation temperature of the flowable dispersion; (h) removing the coated core from the mold cavity; and (i) drying the coated core to remove residual solvent. The mold may be heated to remove solvent, then cooled to set the shell materials.

Another method for making a shell portion by solvent-free molding comprises: (a) melting a thermal reversible carrier, adding and mixing a release-modifying excipient and any other desired ingredients for the shell into the thermal reversible carrier to form a flowable shell material; (b) injecting the flowable shell material (which is heated in a heated feed tank) into a mold cavity (heated to allow the flowable shell material to flow) containing a core such that the flowable shell material surrounds a first portion of the core within the mold cavity; (c) rapidly lowering the temperature of the mold cavity to induce thermal setting of the flowable shell material surrounding the first portion of the core; (d) opening the mold cavity and rotating the portion of the mold containing the core to expose a second portion of the core; (e) closing the mold cavity; (f) injecting heated, flowable shell material into the mold cavity (also heated) such that the flowable shell material surrounds the second portion of the core within the mold cavity; (g) rapidly lowering the temperature of the mold cavity to induce thermal setting of the flowable shell material surrounding the second portion of the core; (h) removing the coated core from the mold cavity. The mold may be optionally rapidly heated or cooled to facilitate removal of dosage form.

In one embodiment, the compression module of United States Patent Publication No. US2003/0072799A1 may be employed to make cores. The shell may then be made applied to these cores using a thermal cycle molding module as described above or by using a zero cycle molding method as described in United States Patent Application No. 10/677,984. A transfer device as described in U.S. Patent Publication No. US2003/0070903 may be used to transfer the cores from the compression module to the thermal cycle molding module. Such a transfer device may have the structure shown as 300 in Figure 3 of United States Patent Publication No. US2003/0068367. It comprises a plurality of transfer units 304 attached in cantilever fashion to a belt 312 as shown in Figures 68 and 69 of United States Patent Publication No. US2003/0068367. The transfer device rotates and operates in sync with the compression module and the thermal cycle molding module to which it is coupled. Transfer units 304 comprise retainers 330 for holding cores as they travel around the transfer device.

In addition to the particular polymeric composition of the present invention as set forth above, other suitable materials for use in or as the flowable material for the core and/or a portion of the shell generally include those comprising thermoplastic materials; film formers; thickeners such as gelling polymers or hydrocolloids; low melting hydrophobic materials such as fats and waxes; non-crystallizable carbohydrates; and the like. Suitable molten components of the flowable material include thermoplastic materials, low melting hydrophobic materials, and the like. Suitable dissolved components for the flowable material include film formers, thickeners such as gelling polymers or hydrocolloids, non-crystallizable carbohydrates, and the like. Suitable dispersed components include insoluble edible materials.

Suitable thermoplastic materials can be molded and shaped when heated, and include both water soluble and water insoluble polymers. Examples of suitable thermoplastic materials include: thermoplastic water swellable cellulose derivatives, thermoplastic water insoluble cellulose derivatives, thermoplastic vinyl polymers, thermoplastic starches, thermoplastic polyalkalene glycols, thermoplastic polyalkylene oxides, and amorphous sugar-glass, and the like, and derivatives, copolymers, and combinations thereof. Examples of suitable thermoplastic water swellable cellulose derivatives include hydroxypropyl cellulose (HPC), hydroxypropylmethyl cellulose (HPMC), methyl cellulose (MC). Examples of suitable water insoluble cellulose derivatives include cellulose acetate (CA), ethyl cellulose (EC), cellulose acetate butyrate (CAB), cellulose propionate. Examples of suitable thermoplastic vinyl polymers include polyvinyl alcohol (PVA) and polyvinyl pyrrolidone (PVP). Examples of suitable thermoplastic starches are disclosed for example in U.S. Patent No. 5,427,614. Examples of suitable thermoplastic polyalkylene glycols include polyethylene glycol. Examples of suitable thermoplastic polyalkylene oxides include polyethylene oxide having a molecular weight from about 100,000 to about 900,000 Daltons. Other suitable thermoplastic materials include sugar in the form on an amorphous glass such as that used to make hard candy forms.

The flowable material for making the core, the shell or a portion thereof, by molding may also optionally comprise adjuvants or excipients, which may comprise, based upon the total wet weight of the flowable material, up to about 30% by weight of the flowable material. Examples of suitable adjuvants or excipients include plasticizers, detackifiers, humectants, surfactants, anti-foaming agents, colorants, flavorants, sweeteners, opacifiers, and the like. Suitable plasticizers for making the core, the shell, or a portion thereof, by molding include, but not limited to the above described plasticizers. In one embodiment, the plasticizer is triethyl citrate. In certain embodiments, the finished, dry shell is substantially free of plasticizers, i.e. contains less than about 1% or less than about 0.01 percent of plasticizers.

In another embodiment, the flowable material comprises, based upon the tota I wet weight of the flowable material, less than about 5% humectants, or alternately is substantially free of humectants, such as glycerin, sorbitol, maltitol, xylitol, or propylene glycol. Humectants have traditionally been included in pre-formed films employed in enrobing processes, such as that disclosed in U.S. Patent Nos. 5,146,730 and 5,459,983, to ensure adequate flexibility or plasticity and bondability of the film during processing. Humectants function by binding water and retaining it in the film. Pre-formed films used in enrobing processes can typically comprise up to 45% water. Disadvantageously, the presence of humectant prolongs the drying process, and can adversely affect the stability of the finished dosage form.

In certain embodiments in which the core, or portions of the shell are prepared using solvent-free molding, the core, or such portions of the shell may comprise active ingredient contained within an excipient matrix. The matrix typically comprises, based upon the total dry weight of the matrix, at least about 30 percent, e.g. at least about 45 weight percent of a thermal-reversible carrier; up to about 30 weight percent of various adjuvants such as for example plasticizers, gelling agents, strengthening agents, colorants, stabilizers, preservatives, and the like as known in the art; and up to about 55 weight percent of one or more release-modifying moldable excipients as described above. In certain embodiments in which a shell portion is prepared by solvent-free molding, and functions to delay the release of one or more active ingredients from an underlying core or core portion, the release modifying excipient may be selected from the swellable, erodible hydrophilic materials. Solvent-free molding may be used to obtain semipermeable, impermeable, or diffusible shell portions.

Examples of suitable thermal-reversible carriers include, but are not limited to Suitable thermal-reversible carriers are thermoplastic materials typically having a melting point below about 110°C, e.g. between about 20°C and about 100°C. Examples of suitable thermal-reversible carriers for solvent-free molding include thermoplastic polyalkylene glycols, thermoplastic polyalkylene oxides, low melting hydrophobic materials, waxes such as edible waxes, e.g. beeswax, thermoplastic polymers, thermoplastic starches, and the like.

Suitable thermoplastic polyalkylene glycols for use as thermal-reversible carriers include polyethylene glycol having molecular weight from about 100 to about 20,000, e.g. from about 100 to about 8,000 Daltons. Suitable thermoplastic polyalkylene oxides include polyethylene oxide having a molecular weight from about 100,000 to about 900,000 Daltons.

Suitable low-melting hydrophobic materials for use as thermal-reversible carriers include fats, fatty acid esters, phospholipids, and waxes which are solid at room temperature, fat-containing mixtures such as chocolate; and the like as disclosed above.

Suitable thermoplastic polymers for use as thermal-reversible carriers include thermoplastic water swellable cellulose derivatives, thermoplastic water insoluble polymers, thermoplastic vinyl polymers, thermoplastic starches, and thermoplastic resins, and combinations thereof. Suitable thermoplastic water swellable cellulose derivatives include hydroxypropylmethyl cellulose (HPMC), methyl cellulose (MC), carboxymethylcellulose (CMC), cross-linked hydroxypropylcellulose, hydroxypropyl cellulose (HPC), hydroxybutylcellulose (HBC), hydroxyethylcellulose (HEC), hydroxypropylethylcellulose, hydroxypropylbutylcellulose, hydroxypropylethylcellulose, and salts, derivatives, copolymers, and combinations thereof. Suitable thermoplastic water insoluble polymers include ethylcellulose, polyvinyl alcohols, polyvinyl acetate, polycaprolactones, cellulose acetate and its derivatives, acrylates, methacrylates, acrylic acid copolymers, and the like and derivatives, copolymers, and combinations thereof. Suitable thermoplastic vinyl polymers include polyvinyl-lacetate, polyvinyl alcohol, and polyvinyl pyrrolidone (PVP).

Examples of suitable thermoplastic starches for use as thermal-reversible carriers are disclosed for example in U.S. Patent No. 5,427,614. Examples of suitable thermoplastic resins for use as thermal-reversible carriers include dammars, mastic, rosin, shellac, sandarac, and glycerol ester of rosin. In one embodiment, the thermal-reversible carrier for making the core, or a portion thereof, by molding is selected from polyalkylene glycols, polyalkylene oxides, and combinations thereof.

The core may be in a variety of different shapes. For example, the core may be shaped as a polyhedron, such as a cube, pyramid, prism, or the like; or may have the geometry of a space figure with some non-flat faces, such as a cone, truncated cone, cylinder, sphere, torus, or the like. In certain embodiments, the core has one or more major faces. For example in embodiments wherein the core is a compressed tablet, the core surface typically has two opposing major faces formed by contact with the upper and lower punch faces in the compression machine. In such embodiments the core surface typically further comprises a "belly-band" located between the two major faces, and formed by contact with the die walls in the compression machine. Exemplary core shapes which may be employed include tablet shapes formed from compression tooling shapes described by "The Elizabeth Companies Tablet Design Training Manual" (Elizabeth Carbide Die Co., Inc., p. 7 (McKeesport, Pa.) as follows (the tablet shape corresponds inversely to the shape of the compression tooling):

18. Shallow Concave.
19. Standard Concave.
20. Deep Concave.
21. Extra Deep Concave.
22. Modified Ball Concave.
23. Standard Concave Bisect.
24. Standard Concave Double Bisect.
25. Standard Concave European Bisect.
26. Standard Concave Partial Bisect.
27. Double Radius.
28. Bevel & Concave.
29. Flat Plain.

30. Flat-Faced-Beveled Edge (F.F.B.E.).
31. F.F.B.E. Bisect.
32. F.F.B.E. Double Bisect.
33. Ring.
34. Dimple.
35. Ellipse.
36. Oval.
37. Capsule.
38. Rectangle.
39. Square.
40. Triangle.
41. Hexagon.
42. Pentagon.
43. Octagon.
44. Diamond.
45. Arrowhead.
46. Bullet.
47. Shallow Concave.
48. Standard Concave.
49. Deep Concave.
50. Extra Deep Concave.
51. Modified Ball Concave.
52. Standard Concave Bisect.
53. Standard Concave Double Bisect.
54. Standard Concave European Bisect.
55. Standard Concave Partial Bisect.
56. Double Radius.
57. Bevel & Concave.
58. Flat Plain.
59. Flat-Faced-Beveled Edge (F.F.B.E.).
60. F.F.B.E. Bisect.
61. F.F.B.E. Double Bisect.
62. Ring.
63. Dimple.
64. Ellipse.
65. Oval.
66. Capsule.
67. Rectangle.
68. Square.
69. Triangle.
70. Hexagon.
71. Pentagon.
72. Octagon.
73. Diamond.
74. Arrowhead.
75. Bullet.
76. Barrel.
77. Half Moon.
78. Shield.
79. Heart.
80. Almond.
81. House/Home Plate.
82. Parallelogram.
83. Trapezoid.
84. Figure 8/Bar Bell.
85. Bow Tie.
86. Uneven Triangle.

In one embodiment of the invention, the core comprises multiple portions, for example a first portion and a second portion. The portions may be prepared by the same or different methods, such as the thermal cycle molding or thermal setting molding methods described herein, and mated using various techniques. For example, the first and second portions may both be made by compression, or both may be made by molding. Or one portion may be made by compression and the other by molding. The same or different active ingredient may be present in the first and second portions of the core. Alternately, one or more core portions may be substantially free of active ingredients. Details of manufacturing cores with multiple portions are well-known in the art and disclosed in, for example, The Theory and Practice of Industrial Pharmacy, 303-306 (3rd Ed. 1986).

In another embodiment of this invention, the core comprises first, second and third portions, each comprising the same or different active ingredient. In another embodiment, the core comprises first, second and third portions, and the third portion (located between the first and second portions) has a higher concentration of active ingredient, thereby causing a particularly desired release profile for at least one active ingredient from the dosage form. In this embodiment, release of at least one active ingredient from the dosage form may have a substantially constant release rate, substantially non-constant release rate, or an ascending release rate.

In another embodiment the core comprises first, second, and third portions, and the third portion (located between the first and second portions) is substantially free of active ingredient, and may contain osmagent or osmopolymer, or may serve as a barrier to the passage of active ingredient between the first and second core portions.

In certain embodiments of the invention, the core or a portion thereof may function to confer modified release properties to at least one active ingredient contained therein. In such embodiments, wherein the core or core portion is made by compression, as previously noted, the core may be comprised of a release-modifying compressible excipient. In such embodiments, wherein the core or core portion is made by molding, as previously noted, the core may be comprised of a release-modifying moldable excipient. In embodiments in which one or more core portions function as an eroding matrix from which dispersed active ingredient is liberated in a sustained, extended, prolonged, or retarded manner, the core portion may be comprised of a release-modifying compressible or moldable excipient selected from swellable erodible hydrophilic agents, pH-dependent polymers, and combinations thereof.

In embodiments in which one or more core portions function as a diffusional matrix through which active ingredient is liberated in a sustained, extended, prolonged, or retarded manner, the core portion may be comprised of a release-modifying excipient selected from combinations of insoluble edible materials and pore formers. Alternately, in such embodiments in which the core portion is prepared by molding, a thermal-reversible carrier may be included in the core portion and may function by dissolving and forming pores or channels through which the active ingredient may be liberated.

As described herein, at least a portion of the shell is semipermeable to a liquid medium. The semipermeable shell or shell portion allows water to be absorbed therethrough and into the core of the dosage form from the environment, such as the dissolution media or gastro-intestinal fluids. The semipermeable shell portion functions as a barrier to the passage of active ingredient from the underlying core portion, forcing the active ingredient to be released from the dosage form via a different avenue, such as an orifice or passageway, or through a diffusible shell portion. The semipermeable shell or shell portions are non-erodible, and they are insoluble in fluids. According to the present invention, at least one portion of such shell that is semipermeable to a liquid medium is comprised of the polymeric composition set forth above.

In one embodiment of this invention, the semipermeable shell or shell portion may be made using any of the flowable materials, including but not limited to the polymeric composition of the present invention, set forth above.

In one embodiment, at least about 30% of the cross-sectional area of the semipermeable shell or semipermeable shell portion used in dosage forms of this invention is non-striated. In other embodiments, at least about 50% of the cross-sectional area of the semipermeable shell or semipermeable shell portion is non-striated. In yet other embodiments, at least about 80% of the cross-sectional area of the semipermeable shell or semipermeable shell portion is non-striated. As used herein, "non-striated" means homogeneous with respect to appearance, and with respect to the internal structure of the shell or shell portion when viewed under any magnification and lighting conditions. For example a cross-section of the shell or shell portion is free of striations, and uniform with respect to refractive properties when observed utilizing a light microscope or a scanning electron microscope at a magnification of about 50 to about 400 times.

The costly and lengthy prior art method for building up a semi-permeable coating on tablets and pharmaceutical dosage forms by spray-coating techniques gives rise to a characteristic striated pattern, which is visible in the cross section of such dosage forms or their semi-permeable coatings as shown in FIGS. 7A and 7B. These characteristic striations are indicative of the spray-coating process consisting of multiple repetitions of the steps consisting of: (a) application via spraying of coating solution; followed by (b) warm air drying, to a tumbling bed of dosage forms in a revolving coating pan such that numerous layers of coating material are built up as each application of coating material dries to form a layer. The thickness of typical sprayed semi-permeable coatings is about 60 to about 150 microns. The thickness of an individual layer is typically in the range of about 10 microns to about 13 microns.

In contrast, the shell or shell portion of the present invention may advantageously be applied to a core directly by a molding process, yielding a uniform and homogeneous layer in 5 minutes or less, e.g. 60 seconds or less, or 30 seconds or less, or 10 seconds or less, and in certain embodiments, say 1 second or less. As such, at least about 30% of the cross-sectional area of the shell or shell portion in certain embodiments of the present invention is non-striated.

Figures 7A and 7B show prior art spray coated compositions having striations which are thus distinguishable from certain embodiments of the present invention. Figure 7A is a micrographic cross-section (121x magnification) of a prior art PROCARDIA XL tablet (commercially available from Pfizer Labs) and Figure 7B is a micrographic cross-section (800x magnification) of the same tablet. This prior art product clearly had striations. In contrast, Figures 8A and 8B show a dosage form of this invention (at 120x and 300x magnifications, respectively). As shown, this embodiment of the invention had no striations.

The shell or shell portion of the present invention has a cross-sectional area, as determined by the equation:

$$S = \pi \left[ (D_1 - D_2)/2 \right]^2$$

Wherein:

$D_1$    is the diameter of the coated dosage form; and
$D_2$    is the diameter of the core of the dosage form,

in the range of about 1 to 900 sq. mm, i.e., e.g., about 25 to 600 sq. mm or about 50 to about 500 sq. mm.

In one particular embodiment, the shell comprises two parts that abut one another, thereby forming a shell that completely surrounds the core.

The shell or shell portion comprised of the above-described polymeric composition of this invention also provides for delivery of active ingredient from the core or core portion to the liquid medium outside the dosage form after the dosage form is contacted with the liquid medium. In one embodiment, this is accomplished by having at least one aperture or passageway within the shell or shell portion to permit liquid medium containing active ingredient within the dosage form to pass through the shell or shell portion and out of the dosage form. In another embodiment, the shell comprises a diffusible shell or shell portion, and active ingredient diffuses therethrough to the liquid medium outside of the dosage form.

In certain other embodiments, a portion of the shell functions as a diffusional membrane which contains pores through which liquid medium containing active ingredient within the dosage form can be released through the diffusible shell portion in a sustained, extended, prolonged or retarded manner. In these embodiments, the rate of release of active ingredient from the underlying core or core portion will depend upon the total pore area in the shell or shell portion, the pathlength of the pores, and the solubility and diffusivity of the active ingredient (in addition to its rate of release from the core or core portion itself). In certain embodiments in which the shell or shell portion functions as a diffusional membrane, the release of the active ingredient from the dosage form may be described as controlled, prolonged, sustained or extended. In these embodiments, the contribution to active ingredient dissolution from the shell or shell portion may follow zero-order, first-order, or square-root of time kinetics. In certain such embodiments, the diffusional membrane shell or shell portion may be comprised of a release-modifying excipient such as a combination of a pore former and an insoluble edible material such as for example a film forming water insoluble polymer. Alternately, in such embodiments in which the shell or shell portion is prepared by solvent-free molding using a thermal reversible carrier, the thermal-reversible carrier may function by dissolving and forming pores or channels through which the active ingredient may be liberated.

In certain other embodiments, a portion of the shell functions as an eroding matrix from which active ingredient dispersed in the shell portion is liberated by the dissolution of successive layers of the shell or shell portion surface. In these embodiments, the rate of active ingredient release will depend on the dissolution rate of the matrix material in the shell or shell portion. Particularly useful matrix materials for providing surface erosion include those which first absorb liquid, then swell and/or gel prior to dissolving. In certain such embodiments, the eroding matrix shell or shell portion may be comprised of a swellable erodible hydrophilic material.

In certain other embodiments, one or more shell portions function as a barrier to prevent release therethrough of an active ingredient contained in the underlying core or core portion. In such embodiments, active ingredient is typically released from a portion of the core which is not covered by the barrier shell portion. Typically, this is achieved by having at least one passageway in the shell or shell portion to permit active ingredient to reach the liquid medium outside the dosage form. Such embodiments advantageously allow for control of the surface area for release of the active ingredient. In certain particular embodiments, for example, the surface area for release of active ingredient can be maintained substantially constant over time. In one embodiment, the release of at least one active ingredient follows substantially zero-order kinetics. In certain such embodiments, the barrier shell portion may be comprised of a water insoluble material such as for example a water insoluble polymer.

In certain other embodiments, the shell or portion thereof functions as a delayed release coating to delay release of an active ingredient which is contained in the core or a portion thereof. In these embodiments, the lag-time for onset of active ingredient release may be governed by erosion of the coating or diffusion through the coating or a combination thereof. In certain such embodiments, the eroding matrix shell or shell portion may be comprised of a swellable erodible hydrophilic material.

In embodiments in which the shell or portion thereof functions to modify the release of an active ingredient which is contained in the core or the subject shell or shell portion, the thickness of the shell or shell portion is critical to the release properties of the dosage form. Advantageously the dosage forms of the invention can be made with precise control over shell thickness. In one embodiment in which the shell or one or more shell portions function to modify the release of an active ingredient which is contained in the core or the subject shell or shell portion, the shell or shell portion is made by the thermal cycle or thermal setting molding methods and apparatus described herein.

In certain other embodiments of the invention, a further degree of flexibility in designing the dosage forms of the present invention can be achieved through the use of an additional outer coating or "overcoating" overlaying the shell or one or more portions thereof. The additional outer coating may be applied for example by spray coating, by compression, or by molding. In such embodiments, the dosage form of the invention comprises at least one active ingredient, which may be the same or different than the active ingredient contained in the core; a core; a shell or shell portion which resides upon at least a portion of the core; and an outer coating which covers at least a portion of the shell or shell portion. The outer coating may for example cover a portion of the first shell portion, or the second shell portion, or both, or may surround the entire shell.

In one embodiment, the outer coating comprises an active ingredient, which is released essentially immediately (i.e. the dissolution of the active ingredient from the outer coating conforms to USP specifications for immediate release dosage forms of the particular active ingredient employed) upon ingestion of the dosage form. After selecting a known method for applying the overcoating to the dosage form, one skilled in the art would then readily know how to prepare a suitable overcoating composition comprised of, for example, up to about 100 mg of an active ingredient and a rapid dissolution material including, but are not limited to, hydroxyalkylcellulose having a molecular weight of less than about 8500.

In another embodiment, the dosage form is a pulsatile drug delivery system, in which one or more shell portions provides for delayed release of a second dose of active ingredient, which is contained in an underlying core portion.

In another embodiment, the dosage form of the present invention may further be coated with, in whole or in part, an overcoating that functions to slow or delay the rate of passage of a fluid, such as water or a biological fluid therethrough. After selecting a known method for applying the overcoating to the dosage form, one skilled in the art would then readily know how to prepare a suitable overcoating composition comprised of, for example, a polymer exhibiting a 8,500 to 4,000,000 molecular weight. Examples of suitable polymers that may be used in the delayed release overcoating include non-ionic water-soluble polymers, cellulose ether nonionic with its solutions unaffected by cations, hydroxyalkylcellulose, hydroxyalkylalkylcellulose, hydroxypropylcellulose, phenylellulose, benzylcellulose, nonionic cellulose ester with its solutions unaffected by cations, benzhydrylcellulose, hydroxyethyloctylcellulose, diphenylmethylcellulose, hydroxyethylcellulose, tritylcellulose and polymer compositions that delay water flux up to about 7.0 hours or up to about 4.5 hours.

In one embodiment of the present invention, in which the shell comprises first and second shell portions, the first and second shell portions may comprise different levels of the same ingredients, e.g. colorants, opacifiers, film-formers, etc. In one such embodiment, the first and second shell portions may be visually distinct from one another, for example the visually distinct portions may be of different colors, hues, glosses, reflective qualities, brightness, depth, shades, chroma, opacity, etc. For example, the shell may have a red portion and a yellow portion, or a flat finish portion and a glossy portion, or an opaque portion and a translucent portion. Alternatively, the first and second shell portions may have different thickness. The first and second shell portions may have different functionalities. For example, the first and second shell portions may confer different release properties to an active ingredient contained in either the subject shell portion, or in a corresponding underlying core portion. In one particular embodiment, the first shell portion may function as a diffusional membrane which contains pores through which fluids can enter the dosage form, and dissolved active ingredient can be released from an underlying core portion; and the second shell portion, may function as an eroding matrix from which active ingredient dispersed in the second shell portion is liberated by the dissolution of successive layers of the shell portion surface.

In one embodiment of this invention, the shell portion or portions of the present invention, whether prepared by a solvent-free molding process or by a solvent-based molding process, are substantially free of pores having a diameter of from about 0.5 microns to about 5.0 microns. As used herein, "substantially free of pores" means that the shell or shell portion or portions have a pore volume of less than about 0.02 cc/g, i.e., e.g., less than about 0.01 cc/g, or less than about 0.005 cc/g in the pore diameter range of about 0.5 microns to about 5.0 microns. In contrast, typical compressed materials have pore volumes of more than about 0.02 cc/g in this diameter range. In another embodiment of this invention, the core is a molded core, and the core or core portions are substantially free of pores having a diameter of from about 0.5 microns to about 5.0 microns.

The total dry weight of the shell or shell portion or portions is about 10 percent to about 400 percent, e.g. from about 10 percent to about 50 percent, of the total dry weight of the core.

Typical shell or shell portion thicknesses that may be employed in this invention are about 10 to about 4000 microns. In certain embodiments, the shell or shell portion has a thickness of less than 800 microns. In embodiments wherein a shell portion is prepared by a solvent-free molding process, the shell portion typically has a thickness of about 500 microns to about 4000 microns, e.g. about 500 microns to about 2000 microns, or about 500 microns to about 800 microns, or about 800 microns to about 1200 microns. In embodiments wherein the shell or shell portion is prepared by a solvent-based molding process, the shell or shell portion typically has a thickness of less than about 800 microns, e.g. about 50 microns to about 500 microns, or about 100 microns to about 350 microns.

In those embodiments in which solvent-free molding is employed, the flowable material may comprise a thermal-reversible carrier as described above.

In embodiments in which the shell portions comprise an active ingredient intended to have immediate release from the dosage form, the shell portion may be prepared via the solvent-free molding method described above. In such embodiments the thermal-reversible carrier may be selected from polyethylene glycol with weight average molecular weight from about 1450 to about 20000, polyethylene oxide with weight average molecular weight from about 100,000 to about 900,000, and the like.

In embodiments in which the shell or shell portion functions to confer modified release properties to at least one active ingredient contained within the dosage form, in the core, the shell or both, the shell or shell portion typically comprises at least one release modifying agent as described above.

In embodiments of the invention in which the core or portion thereof and shell or portion thereof each comprise a dose of active ingredient, the dosage form may function for example as a multi-compartment, e.g. a four-compartment pulsatile release delivery system. In one such embodiment, each of the compartments may comprise a dose of the same active ingredient, to be release at a desired time or rate. In another such embodiment, the corresponding first core portion and first shell portions may comprise a dose of the same first active ingredient to be released at a desired time or rate, while the second core portion and second shell portion may comprise a dose of the same second active ingredient to be released at a desired time or rate. In such embodiments, each compartment comprises inactive materials which enable the desired functionality of that particular core portion or shell portion.

In certain such embodiments, the dosage form may further comprise a water-impermeable barrier layer between the first and second core portions. The water-impermeable barrier layer may be made by any method, for example compression or molding, and comprises at least one water-insoluble material selected from water-insoluble polymers, insoluble edible materials, pH-dependent polymers, and mixtures thereof.

In one particular embodiment of this invention, at least one active ingredient contained within the dosage form exhibits a non-constant release rate.

In another particular embodiment of this invention, at least one active ingredient contained within the dosage form exhibits a delayed burst release profile. By "delayed burst release profile" it is meant that the release of that particular active ingredient from the dosage form is delayed for a pre-determined time after ingestion by the patient, and the delay period ("lag time") is followed by prompt (immediate) release of that active ingredient. At least one shell portion of the present invention provides for the delay period and may be substantially free of the active ingredient to be released in a delayed burst manner. In such embodiments, the delayed burst active ingredient is typically contained within the corresponding underlying core portion. In these embodiments, the core portion may be prepared by compression or molding, and is formulated for immediate release, as is known in the art, so that the core portion is readily soluble upon contact with the dissolution medium. In such embodiments the core portion may comprise a disintegrant, and optionally comprises additional excipients such as fillers or thermoplastic materials selected from water-soluble or low-melting materials, and surfactants or wetting agents. In these embodiments, the dissolution of the burst release active ingredient, after the delay period, meets USP specifications for immediate release tablets containing that active ingredient.

In another particular embodiment of this invention at least one active ingredient contained within the dosage form exhibits a delayed and sustained release profile. By "delayed then sustained release profile" it is meant that the release of that particular active ingredient from the dosage form is delayed for a pre-determined time after ingestion by the patient, and the delay period ("lag time") is followed by sustained (prolonged, extended, or retarded) release of that active ingredient. At least one shell portion of the present invention provides for the delay period, and may be substantially free of the active ingredient to be released in a delayed then sustained manner. In such embodiments, the delayed then sustained release active ingredient may be contained within the corresponding underlying core portion. In such embodiments the core portion may function for example as an eroding matrix or a diffusional matrix, or an osmotic pump. In embodiments in which the core portion functions as a diffusional matrix through which active ingredient is liberated in a sustained, extended, prolonged, or retarded manner, the core portion may comprise a release-modifying excipient, such as for example, insoluble edible materials and/or pore-formers. Alternately, in such embodiments in which the core portion is prepared by molding, the thermal-reversible carrier may function by dissolving and forming pores or channels through which the active ingredient may be liberated. In embodiments in which the core portion functions as an eroding matrix from which dispersed active ingredient is liberated in a sustained, extended, prolonged, or retarded manner, the core portion may be comprised of a release-modifying compressible or moldable excipient, such as, for example, swellable erodible hydrophilic materials and/or pH-dependent polymers.

In embodiments in which one or more core portions function as a diffusional matrix through which active ingredient contained therein is liberated in a sustained, extended, prolonged, or retarded manner, the core portion may be comprised of a release-modifying excipient selected from combinations of insoluble edible materials and pore formers. Alternately, in such embodiments in which the core portion is prepared by solvent-free molding, the thermal-reversible carrier may function by dissolving and forming pores or channels through which the active ingredient may be liberated.

In embodiments in which a shell or shell portion functions by an erosion-based mechanism to provide a time delay for the release of an active ingredient from an underlying core portion, the release-delaying shell or shell portion may be comprised of a release modifying excipient, such as for example, swellable erodible hydrophilic materials and/or insoluble edible materials.

In embodiments of the invention in which a shell or shell portion functions to confer a delay to the release of one or more active ingredients contained in an underlying core portion, the release-delaying shell or shell portion may provide a delay of greater than one hour, for example at least about 3 hours, or at least about 4 hours, or at least about 6 hours, or at least about 12 hours to the onset of dissolution of the active ingredient upon contacting of the dosage form with a liquid medium such as water, gastrointestinal fluid or the like. The delay period is typically controlled by the shell or shell portion thickness, composition, or a combination thereof. In one embodiment the delay period is independent of the pH of the dissolution media or fluid environment. For example, the average lag-time for dissolution of active ingredient in 0.1 N HCl is not substantially different (i.e. within about 30 minutes or within about 15 minutes) from the average lag-time for the dissolution of active ingredient in pH 5.6 buffer system. In certain such embodiments, the release-delaying shell or shell portion may comprise a release modifying excipient, such as for example swellable erodible hydrophilic materials and/or insoluble edible materials.

In embodiments in which the shell or portion thereof confers sustained, extended, or retarded release of an active ingredient contained in an underlying core or core portion, the release-modifying agent in the shell or shell portion may comprise a pore-former, and optionally a film-former. In another embodiment, the shell or shell portion functions as a diffusional membrane. In some such embodiments, the dissolution of the active ingredient may follow "diffusion-controlled" release kinetics, as described for example in Example 1 of U.S. Patent No. 5,286,497. Shells or shell portions which confer sustained, extended, or retarded release and/or function as diffusional membranes can be prepared by a solvent-free method, or a solvent-based method, as described above.

In embodiments in which the shell or portion thereof confers a delayed release to an active ingredient contained in an underlying core or core portion, the release-modifying agent may be selected from swellable erodible hydrophilic materials. The shell portions which confer delayed release can be prepared by a solvent-free method, or a solvent-based method, as described above.

In embodiments in which a shell portion provides a barrier to prevent release therethrough of an active ingredient contained in the underlying core or core portion, the shell portion may be prepared via a solvent-free molding method, as described above. In such embodiments, the thermal-reversible carrier may be selected from, for example, waxes, such as for example carnuba wax, spermaceti wax, beeswax, candelilla wax, shellac wax, microcrystalline wax, and paraffin wax; hydrogenated vegetable oils such as for example cocoa butter, hydrogenated castor oil; other waxy materials such as for example glyceryl behenate, glyceryl palmitostearate, glyceryl monostearate, glyceryl tristearate, glyceryl trilaurylate, glyceryl myristate; thermal-reversible polymers such as for example polycaprolactones and polyvinyl acetate. In certain embodiments, an impermeable barrier can be formed which consists essentially of the thermal reversible carrier. In such embodiments, an additional release-modifying agent is not necessary. In certain other embodiments, the release-modifying agent may be selected from water insoluble polymers such as cellulose acetate, acrylates, acrylic acid copolymers, cellulose acetate, cellulose acetate propionate, cellulose acetate propionate, cellulose propionate, cellulose acetate butyrate, cellulose acetate phthalate, acetaldehyde dimethylcellulose acetate, cellulose acetate ethyl carbamate, cellulose acetate methyl carbamate, cellulose acetate diethyl aminoacetate, ethylcellulose, methacrylates, polyvinyl alcohols, polyvinyl acetate, polycaprolactones, and the like, and mixtures thereof. In such embodiments, the shell or shell portion may optionally further comprise a liquid carrier such as for example mineral oil, propylene glycol, low molecular weight polyethylene glycol, glycerin, and the like.

We have unexpected found that the polymeric composition of the present invention not only possesses superior tensile and yield strength, but it also beneficially withstands high osmotic pressure and hydrodynamic shearing without any disruption, and with minimal distortion. As used herein, "disruption" shall mean a breach or hole in the semipermeable shell portion, resulting in the destruction of the release properties of the dosage form. "Distortion," as used herein, shall mean a stretching of the semipermeable shell film such that the dosage form increases in volume along with a drop in the osmotic pressure, resulting in a slowed release rate that deviates from a substantially zero-order release. Because osmotic dosage forms employ a built-up osmotic pressure inside the dosage form in order to expel the active ingredient through the passageway, it is beneficial for the shell portion to possess such superior tensile and yield strength. We have further found that the incorporation of gelling agents in the semipermeable polymeric composition beneficially contributed to reduce disruption of the osmotic dosage form made therefrom. In addition, we further found that the gelling agents used in the present invention were suitable for use in such polymeric compositions in place of water-soluble materials, the latter of which may tend to contribute to disruption of the osmotic dosage form over an extended period of time.

In addition, both the costs and cycle time associated with manufacturing the osmotic dosage forms using the polymeric composition of the present invention are significantly less than that required by the spray-coated osmotic dosage forms of the prior art.

This invention will be illustrated by the following examples, which are not meant to limit the invention in any way.

Example 1: _Method for Manufacturing the Polymeric Composition_

A polymeric composition suitable for use as a shell for a dosage form and having the formula set forth in Table A below was prepared as follows:

Table A: Formulation of Polymeric Composition

| Ingredient | Trade Name | Manufacturer | Weight %* |
|---|---|---|---|
| Water | -- | --- | 17.17 |
| Acetone | B&J Brand R High Purity Solvent | Honeywell International Inc., Muskegon, MI | 40.08 |
| Cellulose Acetate | Cellulose Acetate, NF | Eastman Chemical Company, Kingsport, TN | 22.90 |
| Carrageenan | Gelcarin GP-812, NF | FMC Corporation, Pharmaceutical Division, Newark, DE | 0.76 |
| Triacetin | Triacetin, Food Grade | Eastman Chemical Company, Kingsport, TN | 15.27 |
| Polyethylene Glycol 400 | Polyethylene Glycol 400 NF, FCC Grade | The Dow Chemical Company, Midland, MI | 3.82 |
| * weight percentage of active ingredient based upon total wet weight of the polymeric composition | | | |

The cellulose acetate was added to a beaker containing acetone, triacetin, polyethylene glycol, and water and mixed using a mixer until all powder was dissolved. The mixture was then heated in the 55°C water bath to obtain a viscous solution. The carrageenan was then added to the hot solution, and the resulting mixture was heated and stirred until a homogeneous texture was obtained.

Example 2: _Method for Manufacturing_ a _Compressed Core_

A core having the formula set forth in Table B below was prepared as follows:

Table B: Formula of Core Portion:

| Ingredient | Trade Name | Manufacturer | Weight % |
|---|---|---|---|
| Polyethylene Oxide (MW=300,000) | Polyox® WSR 750 NF | The Dow Chemical Company, Midland, MI | 75 |
| Diphenhydramine HCl, USP | | Kongo | 15 |
| Hydroxypropyl Methylcellulose | Methocel E5 | Dow Chemical Company, Midland, MI | 8.5 |
| Magnesium stearate | ------ | Mallinckrodt, Inc | 1.5 |
| D&C Yellow # 10 | D&C Yellow #10 HT | Colorcon Inc, Westpoint, Pa | Trace Amount |

(continued)

| Ingredient | Trade Name | Manufacturer | Weight % |
|---|---|---|---|
| Isopropyl Alcohol (dried as solvent) | ------- | EM Science | |

The diphenhydramine HCl, hydroxypropyl methylcellulose, and PEO (MW=300,000) were first mixed in a plastic bag for 1-2 minutes. After this powder mixture was added into a 5 qt. bowl of a Hobart planetary mixer, the alcohol was added thereto with mixing at about 60 rpm. After mixing the ingredients for about 10 minutes, the resulting granulation was removed from the bowl and dried at room temperature for 12 to 16 hours to remove all residual solvent. The granulation was then screened through a #20 mesh screen and put into a plastic bag. Magnesium stearate was added to the dry granules, followed by mixing for 3 minutes.

The resulting diphenhydramine HCl granulation (440 mg) were then fed into the cavity of a model M hydraulic Carver Laboratory Press equipped with a round, concave compression punch and die unit having a 0.4375" diameter.. The granulation was pressed into solid tablets using 2000 lb/sq. in. of compression force. The resulting tablets possessed an approximate density of about 1.1 g/mL.

Example 3: *Method for Manufacturing a Compressed Core Having Two Portions*

Dosage forms comprising a core having a first core portion and a second core portion within a shell were prepared as follows.

The following ingredients were used to make the first core portion (Osmotic layer):

Table C: Formula of Osmotic Core Portion

| Ingredient | Trade Name | Manufacturer | Weight** % |
|---|---|---|---|
| Polyethylene Oxide (MW=7,000,000) | Polyox® WSR Coagulant | The Dow Chemical Company, Midland, MI | 75 |
| Sodium Chloride | | J. T. Baker, Phillipsburg, NJ | 25 |
| FD&C Red #40 | | Warner Jenkinson Company | Trace Amount |
| ** based upon the total dry weight of the osmotic core | | | |

The polyethylene oxide, sodium chloride, and dye were placed in a jar then mixed for about 5 minutes in order to produce the first core portion mixture.

The following ingredients were used to make the second core portion (drug layer):

Table D: Formula of Drug-Containing Core Portion

| Ingredient | Trade Name | Manufacturer | Weight **% |
|---|---|---|---|
| Pseudoephedrine HCl Crystal | | BASF PharmaChemikalien GmbH & Co. | 24.5 |
| Polyethylene Oxide | Polyox® WSR N-80 NF Grade | The Dow Chemical Company, Midland, MI | 75 |
| D&C Yellow # 10 | D&C Yellow #10 HT | Colorcon Inc, Westpoint, Pa | Trace Amount |
| ** based upon the total dry weight of the second core portion | | | |

The polyoxyethylene, pseudoephedrine HCl, and dye were mixed in a jar for about 5 minutes in order to produce the second core portion mixture.

Cores were made from the first and second core portions as described above. The osmotic layer mixture (203 mg) for the first core portion was fed into the cavity mold of a model M hydraulic Carver Laboratory press, equipped with a round, concave compression punch and die unit having a 0.4375" diameter, and then the powder bed was gently tapped. The pseudoephedrine HCl drug layer mixture (425 mg) for the second core portion was then fed into the mold cavity overlying the osmotic core portion. The resulting powder mixture was then pressed into a solid two-portion core using 2000 lb/sq. in. of compression force. The density of the resulting core was approximately 1.1 g/mL.

Example 4: *Method for Manufacturing a Coated Dosage Form Having a Two-Portion Core*

First and second shell portions comprised of the composition made in accordance with Example 1 were applied to a core comprised of the composition made in accordance with Example 3 via a laboratory scale thermal cycle molding unit as disclosed in United States Patent Publication No. US2003/0232082. This molding unit contained a single mold assembly made from an upper mold assembly portion having an upper mold assembly, and a lower mold assembly portion having a lower mold cavity.

After the lower mold assembly portion was cycled to a hot stage at 55.0°C for 30 seconds, approximately 0.2 g of the composition of Example 1 was added to the lower mold cavity. The two-portion core produced in accordance with Example 3 was then inserted into the lower mold cavity such that the second core portion (which contained the drug layer blend) was inserted into the lower mold cavity and the first core portion (which contained the osmotic layer blend) was held tightly by the masking upper mold assembly portion. After the lower mold assembly and upper mold assembly were cycled to a cold stage at 2.0°C for 1 minute, the masking upper mold assembly portion was then removed from the lower mold assembly portion. After the upper mold assembly portion was then cycled to a hot stage at 55.0°C for 30 seconds, approximately 0.2 g of the composition of Example 1 was added to the upper mold cavity. The half-coated core, with the first shell portion, was inserted into the upper mold cavity such that the uncoated first core portion (which contained the osmotic layer blend) rested within the upper mold cavity. The lower mold assembly portion, which had been maintained at 2.0°C, was then mated with the upper mold assembly portion. The upper mold assembly portion was then cycled to a cold stage at 2.0°C for 2 minutes. The lower mold assembly portion was removed and the finished dosage form, a two-portion core coated with a shell comprised of the composition of Example 1, was ejected from the upper mold cavity.

The weight gain due to the shell portion, i.e. the difference in the weight of the finished wet coated dosage form produced in accordance with this Example and the weight of the uncoated dry core produced in accordance with Example 3, was about 30 percent to about 35 percent greater than the weight of the dry core. The finished dosage form was then dried at 50°C for 24 hours to remove all residual solvent. A 0.55 mm aperture was then manually drilled on the pseudophedrine HCl drug layer side of the dosage form by using a pin of diameter of 0.55 mm.

The resulting dosage forms were cut open cross-sectionally, then mounted in a Philips XL 30 ESEM scanning electron microscope. Figure 7A is a micrographic cross-section (121x magnification) of a prior art PROCARDIA XL tablet (commercially available from Pfizer Labs) and Figure 7B is a micrographic cross-section (800x magnification) of the same tablet. As shown in FIGURES 7A and 7B, this prior art product clearly possessed a layered texture as well as striations. In contrast, Figures 8A and 8B show a dosage form of this invention (at 120x and 300x magnifications, respectively) as prepared in accordance with this Example.

This Example showed that the dosage form of the present invention was substantially uniform in texture and had no striations.

Example 5: *Dissolution Testing of Dosage Forms*

The coated dosage forms produced in accordance with Example 4 were analyzed for dissolution using a USP Type II dissolution apparatus (paddies, 50 RPM) and containing a pH 6.8 phosphate buffer media at 37°C. Samples were removed from the apparatus at the following time intervals (hour): 0.25, 0.5, 0.75,1, 1.25, 1.5, 1.75, 2, 2.25, 2.5, 2.75, 3, 4, 5, 6, 7, 8, 9, 10, 11, 12, 13, 14, 15, 16, 17, 18, 19, 20, 21, 22, 23 and 24. These dissolution samples from the dosage forms of Example 4 were analyzed for pseudoephedrine HCl content spectrophotometrically using a Cary 50 UV-Visible spectrophotometer at a wavelength of 254 nm, relative to a pseudoephedrine solution standard prepared at the theoretical concentration for 100% release of the psuedoephedrine. The results were shown in Figure 9. -

This Example showed that the dosage form containing the coating composition of the present invention provided a near zero order release of the pseudoephedrine.

Example 6: *Tensile Testing of Polymeric Composition*

Two polymeric compositions suitable for use as a shell for a dosage form and having the formula set forth in Table E and Table F, respectively, were prepared as follows=

Table E: Cellulose Acetate, Gelcarin, Triacetin, PEG 400

| Ingredient | Trade Name | Manufacturer | Weight % |
|---|---|---|---|
| Cellulose Acetate | Cellulose Acetate, NF | Eastman Chemical Company, Kingsport, TN | 14.74 |

(continued)

| Ingredient | Trade Name | Manufacturer | Weight % |
|---|---|---|---|
| Triacetin | Triacetin, Food Grade | Eastman Chemical Company, Kingsport, TN | 9.83 |
| Polyethylene Glycol 400 | Polyethylene Glycol NF, FCC Grade | The Dow Chemical Company, Midland | 1.23 |
| Carrageenan | Gelcarin GP812 NF | FMC Corporation, Pharmaceutical Division, Newark, DE | 0.49 |
| Acetone | B&J Brand R High Purity Solvent | Honeywell International Inc., Muskegon, MI | 51.60 |
| Water | ----- | ----- | 22.11 |
| * weight percentage of active ingredient based upon total wet weight of the polymeric composition | | | |

The composition set forth in Table E was made in accordance with the procedure set forth in Example 1, but with the use of the components in the amounts set forth in Table E.

Table F: Cellulose Acetate, POLYOX (Comparative Composition)

| Ingredient | Trade Name | Manufacturer | Weight* % |
|---|---|---|---|
| Cellulose Acetate | CA NF 398-10 | Eastman Chemical Company, Kingsport, TN | 80.0 |
| Polyethylene Oxide (MW=200,000) | Polyox® WSR N-80 | Union Carbide Corporation, Danbury, CT | 20.0 |
| Acetone (dried as solvent) | | | |
| * weight percentage of active ingredient based upon total dry weight of the polymeric composition | | | |

The composition set forth in Table F was made under ambient conditions by adding the cellulose acetate to a beaker containing acetone with mixing until all of the powder was dissolved. The polyethylene oxide was then added to the cellulose acetate solution and then mixed until all PEO powder was visually dispersed.

The composition of Table E was then cast into a film. A small amount of the hot molten mixture comprised of the components of Table E was placed on top of a stainless steel plate. Another stainless steel plate, which was pre-chilled to about 5°C, was quickly pressed firmly on top of the mixture for 20 seconds. The solidified film was then removed from the stainless steel plate and dried in a 50 °C oven for 12 hours. This process was then independently repeated, but with the composition of Table F.

Tensile test strips were then created from the films formed from the composition of Table E and the film formed from composition of Table F, respectively, by using a "Punch-Press NAEF" apparatus available from MS Instrument Company Inc., Stony Creek, NY. The length of each respective strip of film was 22+/- 0.25 mm and the width of each respective strip of film was 5 +/- 0.25 mm.

The dry and wet tensile strength of each respective film strip was then analyzed using a TA-xT2i-texture analyzer available from Texture Technologies Corp., Scarsdale, NY. In order to test the wet tensile strength, each respective strip was first submerged in deionized water at room temperature for 2 hours for the film comprised of the composition of Table E and for 1.5 minutes for the film comprised of the composition of Table F. Each respective strip was then removed from the deionized water, blotted dry, and analyzed on the texture analyzer immediately. A strip was individually attached to the grips of the texture analyzer, which were preset at a distance of 10 mm; then the grips traveled at 1 mm/sec during the tensile test. This texture analysis test was repeated for each independent wet strip. As used herein, "hydrated tensile test" shall mean the tensile test performed on wet film that was submerged under water for 2 hours as set forth in this Example.

In order to test the dry tensile strength, additional dry strips were attached to the grips of the texture analyzer, which were preset at a distance of 10 mm, then the grips traveled at 1 mm/sec during the tensile test. The initial thickness of dry film was 0.013 in. for the film comprised of the composition of Table E, and the initial thickness of the dry film was 0.013 in. for the film comprised of the composition of Table F.

The results of the tensile tests performed on the films formed from the compositions set forth in Table E and Table F, respectively, are shown in FIG. 10 and FIG. 11, respectively.

This Example showed that the coating of the present invention, as demonstrated in the film formed from the composition of Table E, possessed superior wet tensile strength in comparison to that possessed by coatings devoid of gelling agents and coatings formed via organic dispersion, as demonstrated in the film formed from the composition of Table F.

This Example further showed that the tensile strength of the film in wet form changed as a function of wetting time. More specifically, the wet film formed from the composition of Table F became significantly weaker over time, relative to the same dry film. However, the tensile strength of the wet film formed from the composition of Table E surprising exhibited increased tensile strength over time, relative to the tensile strength of the same dry film.

Example 7: *Tensile Testing of Polymeric Composition*

Two polymeric compositions suitable for use as a shell for a dosage form and having the formula set forth in Table G and Table H, respectively, were prepared as follows:

Table G: Cellulose Acetate, Triacetin (high level), made from acetone/water

| Ingredient | Trade Name | Manufacturer | Weight % |
|---|---|---|---|
| Cellulose Acetate | Cellulose Acetate, NF | Eastman Chemical Company, Kingsport, TN | 18.83 |
| Triacetin | Triacetin, Food Grade | Eastman Chemical Company, Kingsport, TN | 22.60 |
| Carrageenan | Gelcarin GP812 NF | FMC Corporation, Pharmaceutical Division, Newark, DE | 2.07 |
| Acetone (dried as solvent) | B&J Brand R High Purity Solvent | Honeywell International Inc., Muskegon, MI | 39.55 |
| Water (dried as solvent) | ----- | ----- | 16.95 |
| * weight percentage of active ingredient based upon total wet weight of the polymeric composition | | | |

Table H: Cellulose Acetate, Triacetin (low level), made from acetone/water.

| Ingredient | Trade Name | Manufacturer | Weight %* |
|---|---|---|---|
| Cellulose Acetate | Cellulose Acetate, NF | Eastman Chemical Company, Kingsport, TN | 21.37 |
| Triacetin | Triacetin, Food Grade | Eastman Chemical Company, Kingsport, TN | 12.82 |
| Carrageenan | Gelcarin GP812 NF | FMC Corporation, Pharmaceutical Division, Newark, DE | 1.71 |
| Acetone (dried as solvent) | B&J Brand R High Purity Solvent | Honeywell International Inc., Muskegon, MI | 44.87 |
| Water (dried as solvent) | ----- | ----- | 19.23 |
| * weight percentage of active ingredient based upon total wet weight of the polymeric composition | | | |

The compositions set forth in Table G and Table H, respectively, were made in accordance with the procedure set forth in Example 1, but with the omission of the PEG 400 and with the compounds in the amounts set forth in Table G and Table H, respectively.

The composition of Table G and the composition of Table H were then independently casted into films in accordance with the procedure set forth in Example 6. Tensile test strips were then created from the film formed from the composition of Table G and the film formed from composition H, independently, via the procedure set forth in Example 6. The dry and wet tensile strength of each respective film strip was then analyzed via the procedure set forth in Example 6 except: a) the test strips were submerged in deionized water for 3 minutes; b) the grips were set at 22 mm; and c) the grips traveled at a speed of 0.1 mm/sec.

The initial thickness of dry film was 0.005 in. for the film comprised of the composition of Table G and H, respectively, and the thickness of the wet film immediately after wetting for 3 minutes comprised of the composition of Table G and H, respectively, was 0.006 in.

The results of the tensile tests performed on the films formed from the compositions set forth in Table G, and Table H, respectively, are shown in FIG. 12, and FIG. 13, respectively.

This Example showed that, for both wet films and dry films, the addition of an excess amount of plasticizers may increase the films' flexibility but may also reduce the films' tensile strength.

Example 8: *Method for Manufacturing a Coated Dosage Form Having a Two-Portion Core*

The shell portions of a dosage form are comprised of the composition set forth in Example 1, and the core is comprised of the composition set forth in Example 3.

A molding unit similar to that of Example 4 contains a single mold assembly with: a) an upper mold assembly portion having an upper mold cavity;and b) a lower mold assembly portion having a lower mold cavity; however, this unit also has injection ports into each cavity of each respective upper and lower mold assembly. A two-portion core as set forth in Example 3 is then inserted into the lower mold cavity of the molding unit, which is maintained at 5 °C such that the second core portion (which contains the drug layer blend) is inserted into the lower mold cavity and the first core portion (which contains the osmotic layer blend) is held tightly by the masking upper mold assembly portion. Approximately 0.2 g of the composition of Example 1 is injected into the lower mold cavity via an injection port The masking upper mold assembly portion is then removed from the lower mold assembly portion. The upper mold assembly portion, which is kept at 5 °C, is then mated with the lower mold assembly portion. Approximately 0.2 g of the composition of Example 1 is injected into the upper mold cavity via an injection port. The lower mold assembly portion is removed and the finished dosage form, a two-portion core coated with a shell comprised of the composition of Example 1, is ejected from the upper mold cavity.

**Claims**

1.  A dosage form comprising:

    (a) at least one active ingredient;
    (b) a core having an outer surface; and
    (c) a shell which resides upon at least a portion of the core outer surface, wherein at least a portion of the shell is semipermeable and is comprised of, based upon the total dry weight of the shell, from about 40 percent to about 99 percent of a water insoluble film forming polymer and from about 1 percent to about 30 percent of a gelling agent, and the shell comprises means for providing the active ingredient to a liquid medium outside the shell after contacting of the dosage form with the liquid medium.

2.  The dosage form of claim 1, wherein the water insoluble, film forming polymer is comprised of at least one of the following: cellulose esters, cellulose ethers, cellulose ester-ethers, polyvinyl alcohols, polyvinyl acetate, polycaprolactones, polyacrylate, polymethacrylate, or derivatives or copolymers thereof.

3.  The dosage form of claim 1, wherein the water insoluble film forming polymer is cellulose acetate and/or ethyl cellulose.

4.  The dosage form of claim 3, wherein the gelling agent is comprised of at least one of the following: hydrocolloids, gelling starches, or derivatives or copolymers thereof.

5.  The dosage form of claim 4, wherein the gelling agent is a hydrocolloid selected from the group consisting of carrageenan, gellan gum, locust bean gum, agar, alginate, pectin, and mixtures thereof.

6.  The dosage form of claim 1, wherein the shell has at least one passageway extending from the surface of the shell, through the shell, and to at least the surface of the core.

7.  The dosage form of claim 1, wherein the shell comprises a first shell portion which is semipermeable to a liquid medium and comprised of, based upon the total dry weight of the shell, from about 40 percent to about 99 percent of a water insoluble film forming polymer and from about 1 percent to about 30 percent of a gelling agent, and a second shell portion which is compositionally different than the first shell portion, the first and second shell portions each are substantially in contact with the core outer surface.

8. The dosage form of claim 7, in which at least one of the first or second shell portions has at least one passageway therein extending from the surface of the shell portion, through the shell, and to at least the surface of the core.

9. The dosage form of claim 1 or claim 7, wherein the shell and core have a continuous cavity therein defining an interior surface, wherein the shell does not extend substantially upon the interior surface.

10. The dosage form of claim 1 or claim 7, wherein said core comprises a first core portion, a second core portion, and a third core portion located between the first and second core portions, wherein the third core portion comprises an osmopolymer.

11. The dosage form of claim 1 or claim 7, in which the core comprises, based upon the total weight of the core, from about 0.01 percent to about 40 percent of an osmagent selected from the group consisting of sodium chloride, magnesium sulfate, magnesium chloride, potassium sulfate, sodium sulfate, lithium sulfate, potassium hydrogen phosphate, mannitol, urea, inositol, magnesium succinate, tartaric acid, carbohydrate, and mixtures thereof

12. The dosage form of claim 1 or claim 7, wherein at least a portion of the shell is further comprised of, based upon the total dry weight of the shell, from about 0.5 percent to about 5 percent of a pore former selected from the group consisting of polyethylene glycol, hydroxypropylmethylcellulose, hydroxypropylcellulose, and copolymers and mixtures thereof.

13. The dosage form of claim 1 or claim 7, wherein at least a portion of the shell is further comprised of, based upon the total dry weight of the shell, from about 1 percent to about 30 percent of a plasticizer.

14. The dosage from of claim 13, wherein the plasticizer is selected from the group consisting of polyethylene glycol; propylene glycol; glycerin; sorbitol; triethyl citrate; tributyl citrate; dibutyl sebecate; vegetable oils; polysorbates; sodium lauryl sulfates; dioctyl-sodium sulfosuccinates; mono acetate of glycerol; diacetate of glycerol; triacetate of glycerol; natural gums; triacetin; acetyltributyl citrate; diethyloxalate; diethylmalate; diethyl fumarate; diethylmalonate; dioctylphthalate; dibutylsuccinate; glycerol tributyrate; hydrogenated castor oil; fatty acids; glycerides; and mixtures thereof.

15. A composition comprising, based upon the total wet weight of the composition:

(a) from about 5 percent to about 50 percent of a water insoluble, film forming polymer;
(b) from about 0.05 percent to about 15 percent of a gelling agent; and
(c) from about 40 percent to about 95 percent of a multisolvent system, wherein the multisolvent system is comprised of, based upon the total weight of the multisolvent system, from about 10 percent to about 50 percent of water and from about 50 percent to about 90 percent of a water miscible organic solvent.

**Patentansprüche**

1. Dosierungsform, umfassend:

(a) wenigstens einen wirksamen Inhaltsstoff;
(b) einen Kern mit einer Außenfläche; und
(c) einen Mantel, der über wenigstens einem Teil der Kernaußenfläche liegt, wobei wenigstens ein Abschnitt des Mantels halbdurchlässig ist und, bezogen auf das Gesamttrockengewicht des Mantels, von etwa 40 Prozent bis etwa 90 Prozent eines wasserunlöslichen, filmbildenden Polymers und von 1 Prozent bis etwa 30 Prozent eines Gelierungsmittels umfasst und der Mantel Mittel zur Abgabe des aktiven Inhaltsstoffes an ein flüssiges Medium außerhalb des Mantels nach Inkontaktbringen der Dosierungsform mit dem flüssigen Medium umfasst.

2. Dosierungsform nach Anspruch 1, wobei das wasserunlösliche, filmbildende Polymer wenigstens eine der folgenden Verbindungen umfasst: Celluloseester, Celluloseether, Celluloseesterether, Polyvinylalkohole, Polyvinylacetat, Polycaprolactone, Polyacrylat, Polymethacrylat oder Derivate oder Copolymere davon.

3. Dosierungsform nach Anspruch 1, wobei das wasserunlösliche, filmbildende Polymer Celluloseacetat und/oder Ethylcellulose ist.

4. Dosierungsform nach Anspruch 3, wobei das Gelierungsmittel wenigstens eine der folgenden Verbindungen umfasst: Hydrokolloide, gelierende Stärken oder Derivate oder Copolymere davon.

5. Dosierungsform nach Anspruch 4, wobei das Gelierungsmittel ein Hydrokolloid ist, das ausgewählt ist aus der Gruppe, bestehend aus Carrageenan, Gellangummi, Johannesbrotgummi, Agar, Alginat, Pectin und Mischungen davon.

6. Dosierungsform nach Anspruch 1, wobei der Mantel wenigstens einen Durchlass aufweist, der sich von der Oberfläche des Mantels, durch den Mantel hindurch und bis wenigstens zur Oberfläche des Kerns erstreckt.

7. Dosierungsform nach Anspruch 1, wobei der Mantel einen ersten Mantelabschnitt, der für ein flüssiges Medium halbdurchlässig ist und, bezogen auf das Gesamttrockengewicht des Mantels, von etwa 40 Prozent bis etwa 99 Prozent eines wasserunlöslichen, filmbildenden Polymers und von etwa 1 Prozent bis etwa 30 Prozent eines Gelierungsmittels umfasst, und einen zweiten Mantelabschnitt, der im Hinblick auf die Zusammensetzung verschieden ist von dem ersten Mantelabschnitt, umfasst, wobei die ersten und zweiten Mantelabschnitte jeweils im wesentlichen in Kontakt mit der Kernaußenfläche stehen.

8. Dosierungsform nach Anspruch 7, bei der wenigstens einer der ersten oder zweiten Mantelabschnitte wenigstens einen Durchlass darin aufweist, der sich von der Oberfläche des Mantelabschnittes, durch den Mantel hindurch und bis zu wenigstens der Oberfläche des Kerns erstreckt.

9. Dosierungsform nach Anspruch 1 oder Anspruch 7, wobei der Mantel und Kern einen kontinuierlichen Hohlraum aufweisen, der darin eine Innenfläche definiert, wobei der Mantel sich nicht wesentlich auf die Innenfläche erstreckt.

10. Dosierungsform nach Anspruch 1 oder Anspruch 7, wobei der Kern einen ersten Kernabschnitt, einen zweiten Kernabschnitt und einen dritten Kernabschnitt, der zwischen den ersten und zweiten Kernabschnitten angeordnet ist, umfasst, wobei der dritte Kernabschnitt ein Osmopolymer umfasst.

11. Dosierungsform nach Anspruch 1 oder Anspruch 7, bei der der Kern, bezogen auf das Gesamtgewicht des Kerns, von etwa 0,01 Prozent bis etwa 40 Prozent eines osmotischen Mittels umfasst, das ausgewählt ist aus der Gruppe, bestehend aus Natriumchlorid, Magnesiumsulfat, Magnesiumchlorid, Kaliumsulfat, Natriumsulfat, Lithiumsulfat, Kaliumhydrogenphosphat, Mannitol, Harnstoff, Inositol, Magnesiumsuccinat, Weinsäure, Kohlehydrat und Mischungen davon.

12. Dosierungsform nach Anspruch 1 oder Anspruch 7, wobei wenigstens ein Abschnitt des Mantels, bezogen auf das Gesamttrockengewicht des Mantels, außerdem von etwa 0,5 Prozent bis etwa 5 Prozent eines Porenbildners umfasst, der ausgewählt ist aus der Gruppe, bestehend aus Polyethylenglykol, Hydroxypropylmethylcellulose, Hydroxypropylcellulose und Copolymeren und Mischungen davon.

13. Dosierungsform nach Anspruch 1 oder Anspruch 7, wobei wenigstens ein Abschnitt des Mantels, bezogen auf das Gesamttrockengewicht des Mantels, außerdem von etwa 1 Prozent bis etwa 30 Prozent eines Weichmachers umfasst.

14. Dosierungsform nach Anspruch 13, wobei der Weichmacher ausgewählt ist aus der Gruppe, bestehend aus Polyethylenglykol; Propylenglykol; Glycerin; Sorbitol; Triethylcitrat; Tributylcitrat; Dibutylsebacat; pflanzlichen Ölen; Polysorbaten; Natriumlaurylsulfaten; Dioctylnatriumsulfosuccinaten; Monoacetat von Glycerol; Diacetat von Glycerol; Triacetat von Glycerol; natürlichen Gummis; Triacetin; Acetyltributylcitrat; Diethyloxalat; Diethylmalat; Diethylfumarat; Diethylmalonat; Dioctylphthalat; Dibutylsuccinat; Glyceroltributyrat; hydriertem Rizinusöl; Fettsäuren; Glyceriden; und Mischungen davon.

15. Zusammensetzung, die, bezogen auf das Gesamtnassgewicht der Zusammensetzung:

   (a) von etwa 5 Prozent bis etwa 50 Prozent eines wasserunlöslichen, filmbildenden Polymers;
   (b) von etwa 0,05 Prozent bis etwa 15 Prozent eines Gelierungsmittels; und
   (c) von etwa 40 Prozent bis etwa 95 Prozent eines Mehrlösemittelsystems umfasst, wobei das Mehrlösemittelsystem, bezogen auf das Gesamtgewicht des Mehrlösemittelsystems, von etwa 10 Prozent bis etwa 50 Prozent Wasser und von etwa 50 Prozent bis etwa 90 Prozent eines wassermischbaren organischen Lösemittels umfasst.

**Revendications**

1.  Forme posologique comprenant :

    (a) au moins un ingrédient actif ;
    (b) un noyau présentant une surface extérieure ; et
    (c) une enveloppe disposée sur au moins une partie de la surface extérieure du noyau, au moins une partie de l'enveloppe étant semi-perméable et constituée, sur la base du poids sec total de l'enveloppe, d'environ 40 % à environ 99 % d'un polymère filmogène insoluble dans l'eau et d'environ 1 % à environ 30 % d'un agent gélifiant, l'enveloppe comprenant des moyens permettant la diffusion de l'ingrédient actif vers un milieu liquide situé à l'extérieur de l'enveloppe après contact de la forme posologique avec le milieu liquide.

2.  Forme posologique selon la revendication 1, dans laquelle le polymère filmogène insoluble dans l'eau est constitué d'au moins un des composés suivants : esters de cellulose, éthers de cellulose, ester-éthers de cellulose, alcools polyvinyliques, polyacétate de vinyle, polycaprolactones, polyacrylate, polyméthacrylate, ou dérivés ou copolymères de ceux-ci.

3.  Forme posologique selon la revendication 1, dans laquelle le polymère filmogène insoluble dans l'eau est l'acétate de cellulose et/ou l'éthylcellulose.

4.  Forme posologique selon la revendication 3, dans laquelle l'agent gélifiant est constitué d'au moins un des composés suivants : hydrocolloïdes, amidons gélifiants, ou dérivés ou copolymères de ceux-ci.

5.  Forme posologique selon la revendication 4, dans laquelle l'agent gélifiant est un hydrocolloïde choisi dans le groupe constitué du carraghénane, de la gomme gellane, de la gomme de caroube, de l'agar-agar, de l'alginate, de la pectine, et de leurs mélanges.

6.  Forme posologique selon la revendication 1, dans laquelle l'enveloppe possède au moins un passage s'étendant depuis la surface de l'enveloppe, traversant l'enveloppe, et allant au moins jusqu'à la surface du noyau.

7.  Forme posologique selon la revendication 1, dans laquelle l'enveloppe comprend une première partie d'enveloppe qui est semi-perméable à un milieu liquide et constituée, sur la base du poids sec total de l'enveloppe, d'environ 40 % à environ 99 % d'un polymère filmogène insoluble dans l'eau et d'environ 1 % à environ 30 % d'un agent gélifiant, et une seconde partie d'enveloppe qui a une composition différente de celle de la première partie d'enveloppe, les première et seconde parties de l'enveloppe étant chacune substantiellement en contact avec la surface extérieure du noyau.

8.  Forme posologique selon la revendication 7, dans laquelle au moins une des première ou seconde parties d'enveloppe possède au moins un passage en elle s'étendant depuis la surface de la partie d'enveloppe, traversant l'enveloppe, et allant au moins jusqu'à la surface du noyau.

9.  Forme posologique selon la revendication 1 ou la revendication 7, dans laquelle l'enveloppe et le noyau ont une cavité continue en eux délimitant une surface intérieure, l'enveloppe ne s'étendant pas substantiellement sur la surface intérieure.

10. Forme posologique selon la revendication 1 ou la revendication 7, dans laquelle ledit noyau comprend une première partie de noyau, une deuxième partie de noyau, et une troisième partie de noyau située entre la première et la deuxième partie du noyau, la troisième partie du noyau comprenant un osmopolymère.

11. Forme posologique selon la revendication 1 ou la revendication 7, dans laquelle le noyau comprend, sur la base du poids total du noyau, environ 0,01 % à environ 40 % d'un osmoagent choisi dans le groupe constitué du chlorure de sodium, du sulfate de magnésium, du chlorure de magnésium, du sulfate de potassium, du sulfate de sodium, du sulfate de lithium, de l'hydrogéno-phosphate de potassium, du mannitol, de l'urée, de l'inositol, du succinate de magnésium, de l'acide tartrique, d'un glucide, et de leurs mélanges.

**12.** Forme posologique selon la revendication 1 ou la revendication 7, dans laquelle au moins une partie de l'enveloppe est constituée en outre, sur la base du poids sec total de l'enveloppe, d'environ 0,5 % à environ 5 % d'une substance porogène choisie dans le groupe constitué du polyéthylèneglycol, de l'hydroxypropylméthylcellulose, de l'hydroxypropylcellulose, et de leurs copolymères et mélanges.

**13.** Forme posologique selon la revendication 1 ou la revendication 7, dans laquelle au moins une partie de l'enveloppe est constituée en outre, sur la base du poids sec total de l'enveloppe, d'environ 1 % à environ 30 % d'un plastifiant.

**14.** Forme posologique selon la revendication 13, dans laquelle le plastifiant est choisi dans le groupe constitué du polyéthylèneglycol, du propylèneglycol, de la glycérine, du sorbitol, du citrate de triéthyle, du citrate de tributyle, du sébaçate de dibutyle, des huiles végétales, des polysorbates, des laurylsulfates de sodium, des dioctylsulfosuccinates de sodium, du monoacétate de glycérol, du diacétate de glycérol, du tri-acétate de glycérol, des gommes naturelles, de la triacétine, du citrate d'acétyltributyle, de l'oxalate de diéthyle, du malate de diéthyle, du fumarate de diéthyle, du malonate de diéthyle, du phtalate de dioctyle, du succinate de dibutyle, du tributyrate de glycérol, de l'huile de ricin hydrogénée, des acides gras, des glycérides, et des mélanges de ceux-ci.

**15.** Composition comprenant, sur la base du poids humide total de la composition :

(a) environ 5 % à environ 50 % d'un polymère filmogène insoluble dans l'eau ;
(b) environ 0,05 % à environ 15 % d'un agent gélifiant ; et
(c) environ 40 % à environ 95 % d'un système à plusieurs solvants, ledit système étant constitué, sur la base du poids total du système à plusieurs solvants, d'environ 10 % à environ 50 % d'eau et d'environ 50 % à environ 90 % d'un solvant organique miscible à l'eau.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 3845770 A
- US 3995631 A
- US 4111202 A
- US 4327725 A
- US 4449983 A
- US 4627971 A
- US 4892739 A
- US 5830501 A
- US 6342249 B
- EP 0384642 A
- US 4576604 A
- US 3173876 A
- US 3276586 A
- US 4541005 A
- US 3541006 A
- US 3546142 A
- US 3133132 A
- US 3916899 A
- US 4160020 A
- US 4906478 A
- US 5275822 A
- US 6103260 A
- US 4173626 A
- US 4863742 A
- US 4980170 A
- US 4984240 A
- US 5286497 A
- US 5912013 A
- US 6270805 A
- US 6322819 A
- US 20030072799 A
- US 20030124183 A
- US 20030086973 A1
- US 20030068367 A
- US 20030072799 A1
- US 10677984 B
- US 20030070903 A
- US 5427614 A
- US 5146730 A
- US 5459983 A
- US 20030232082 A

**Non-patent literature cited in the description**

- **Verma et al.** Osmotically Controlled Oral Drug Delivery. *Drug Development and Industrial Pharmacy,* 2000, vol. 26 (7), 695-708
- **Maffione et al.** High-Viscosity HPMC as a Film-Coating Agent. *Drug Development and Industrial Pharmacy,* 1993, vol. 19 (16), 2043-2053
- **Remington.** *The Science and Practice of Pharmacy,* 2000, 208-209
- **Scott ; Roff.** Handbook of Common Polymers. 1971, 72-8198, 281
- **Ratner ; Hoffman.** ACS Symposium Series. American Chemical Society, 1976, 1-36
- **Schacht.** *Recent Advances in Drug Delivery Systems,* 1984, 259-278
- **Lachman et al.** The Theory and Practice of Industrial Pharmacy. 1986
- The Theory and Practice of Industrial Pharmacy. 1986, 303-306